# EUROPEAN PATENT APPLICATION

(11) **EP 3 537 154 A2**
(43) Date of publication of application: **11.09.2019**
(21) Application number: 19161183.9
(22) Date of filing: 07.03.2019
(51) Int. Cl.: G01N 33/574, G01N 33/68

(54) **SRM/MRM ASSAYS FOR PROFILING TUMOR TISSUE**

(30) Priority: 09.03.2018 US 201862640632 P
(71) Applicant: NantOmics, LLC, Culver City, CA 90232 (US)
(72) Inventor: Hembrough, Todd, Culver City, CA 90232 (US); Cecchi, Fabiola, Culver City, CA 90232 (US); Schwartz, Sarit, Culver City, CA 90232 (US); Scott, Kerry, Culver City, CA 90232 (US)
(74) Representative: AWA Sweden AB

(57) **Abstract**

Methods are provided herein for using SRM/MRM assays to detect and quantitate proteins involved in all cellular processes including cell division, cellular differentiation, cell growth inhibition, cellular metabolism, cell signaling, and tumor immune response/modulation in a protein digest prepared from a biological sample of formalin fixed tumor tissue. The SRM/MRM assays can provide a tumor tissue profile of the entire tissue microenvironment, regardless of cellular origin of expression, which can provide an optimal cancer therapy treatment.

## Description

### FIELD

Methods are provided for mass spectrometry-based quantitative proteomic analysis of tumor tissue obtained from a cancer patient for a personalized patient tumor profile. These protein assays performed on patient tumor tissue can detect and quantitate levels of proteins that: 1) associate with a positive or negative response to immuno-based cancer therapy, where the assays can effectively predict a positive or negative response to immuno-based cancer therapy, 2) associate with a positive or negative response to molecularly-targeted cancer therapy, where the assays can predict a positive or negative response to molecularly-targeted cancer therapy, 3) initiate, inhibit, maintain, promote, and/or otherwise modulate the patient's own immune system to attack the patient's own tumor cells. The cancer patient tumor tissue proteomic profile provided by these methods is used as part of improved cancer treatment methods that employ cancer therapeutic agents designed either to specifically attack and kill tumor cells or to manipulate the patient tumor immune response to kill tumor cells.

### BACKGROUND

Small molecule and biological inhibitors of proteins (cancer biomarkers) that are aberrantly expressed in tumor cells and that drive tumor cell growth and survival are used as therapeutic agents against a variety of cancers. Examples of cancer biomarker proteins that are aberrantly expressed and that drive tumor cell growth and survival include Met, IGF-1R, and Her2. Cancer therapeutic agents have been developed to interact directly with these proteins and, accordingly, detecting expression of and quantifying these proteins directly in patient tumor tissue can be used as part of a treatment regimen to select and administer a therapeutic agent, or combination of agents, that will inhibit function of these proteins, preventing tumor cell growth and promoting overall patient survival. Precise detection and quantitation of proteins in tumor tissue, and especially in tumor cells present in such tissue, can be effectively performed via mass spectrometry-SRM/MRM analysis of specific protease-digested peptides derived from expressed proteins in tumor cells extracted from patient tumor tissue by tissue microdissection. Quantifying such groups of proteins at one time in a single SRM/MRM assay provides for a "profile" or "signature" of the protein expression landscape for informing tumor cell-targeted cancer therapeutics. Examples of quantitative assays that can be used as part of treatment regimens with targeted therapeutic agents include IGF-1R protein (see U.S. Patent 8,728,753) and cMet protein (see U.S. Patent 9,372,195).

A further example of a cancer therapeutic agent designed to specifically inhibit the function of proteins that drive the cancer process, and thus prevent tumor cell growth, is trastuzumab. Trastuzumab, sold under the brand name Herceptin® among others, is a monoclonal antibody used primarily to treat breast cancer, but also other cancers, that express the Her2 receptor protein in tumor cells within the tumor tissue. Trastuzumab binds to domain IV of the extracellular segment of the Her2 receptor protein. Tumor cells treated with trastuzumab undergo arrest during the G1 phase of the cell cycle so there is reduced proliferation. It has been suggested that trastuzumab does not alter Her-2 gene expression, but downregulates activation of AKT. In addition, trastuzumab suppresses angiogenesis both by induction of antiangiogenic factors and repression of proangiogenic factors. It is thought that a contribution to the unregulated growth observed in cancer could be due to proteolytic cleavage of Her2 protein that results in the release of the extracellular domain. Trastuzumab has also been shown to inhibit Her2 ectodomain cleavage in breast cancer cells, which also helps to inhibit tumor cell growth.

Proteins that characterize the cancer patient tumor immune response are being studied by the pharmaceutical industry. These proteins are expressed normally, and aberrantly, in many different cell types, including tumor cells, benign cells in solid tissue, and the various lineages of blood-born lymphocytes. These proteins function to initiate, enhance, modulate, or inhibit a patient's own immune response to his/her own tumor cells. While each of these proteins has a distinct function, their effect on the immune system can depend upon which cell is expressing the protein. In the normal setting, the immune system functions to eradicate tumor cells through a complex molecular signaling process of self vs. non-self-recognition mediated through lymphocyte-dependent tumor cell killing. However, this complex process can be disrupted by tumor cells that seek to evade immune surveillance. Small molecule and biological therapeutic agents have been developed that interact with these proteins in order to manipulate the immune system to attack and kill tumor cells. Successful administration of targeted immunomodulatory therapeutic agents would greatly benefit from a protein expression "profile" or "signature" of the patient immune system landscape in order to determine which target proteins are expressed within the tissue. This immune profile can then inform which therapeutic agent, or combination of agents, would most likely provide the greatest chance of arming, modulating, manipulating, and/or supporting the patient immune system against tumor cells for optimal patient outcome.

An example of a type of cancer therapeutic agent designed to manipulate the patient immune system is a collection of compounds known as immune checkpoint inhibitors that interact with the collection of proteins known as immune checkpoint proteins. The PD-1 protein is an immune checkpoint protein that normally resides on lymphocytes called T cells, acting as a type of "off switch" that helps keep the T cells from attacking other cells in the body. It does this when it attaches to PD-L1, a protein present on the surface of some normal (and cancer) cells. When PD-1 binds to PD-L1, it signals the T cell to not attack the cell expressing PD-L1. Some cancer cells have large amounts of PD-L1, which masks them to the immune system allowing them to evade immune surveillance preventing an attack from T cells. Monoclonal antibodies that target either PD-1 or PD-L1 can unmask the cancer cells boosting the immune response against cancer cells. This cancer treatment strategy has shown a great deal of promise in treating certain cancers and examples of PD-1 inhibitors include pembrolizumab (Keytruda®) and nivolumab (Opdivo®). These drugs have shown to be helpful in treating several types of cancer, including melanoma, non-small cell lung cancer, kidney cancer, head and neck cancers, and Hodgkin's lymphoma. They also are being studied for use against many other types of cancer. An example of a PD-L1 inhibitor is atezolizumab (Tecentriq®) which currently is used to treat bladder cancer, and is also being studied for use against other types of cancer. Many other drugs that target either PD-1 or PD-L1 are also now being tested in clinical trials, both alone and combined with other drugs. These examples demonstrate how knowledge of the molecular status of both the tumor cells and immune system cells can be used as part of an effective targeted immune-based cancer treatment strategy.

The ability to procure homogenous populations of cells directly from patient tumor tissue sections for molecular profiling of histologically-specified cell populations is highly advantageous whereby, for example, tumor cells that may reside within other types of cells including normal epithelial cells, endothelial cells, fibroblasts, and immune cells can be studied. Laser Capture Microdissection (LCM) technology (see U.S. Patent 6,867,038) can be used to compartmentalize molecular analysis of tumor tissue to precisely defined populations of cells. LCM, as well as other commercially available tissue microdissection technologies, including DIRECTOR® technology (see U.S. Patent 7,381,440), has improved the analysis of tissue samples by allowing molecular profiling of cells derived from tissue samples to be placed in a pathologically relevant context.

### SUMMARY

Therefore, provided herein are new SRM/MRM assays that may be used to create a protein expression "profile" or "signature of the patient immune system landscape. Specifically the SRM/MRM assays can be used to detect and quantitate levels of specific proteins in proteomic lysates prepared directly from cancer patient tumor tissue. These proteins can be used to inform choice of cancer therapeutics and as part of a treatment regimen. The proteins that are analyzed provide for the initiation and growth of tumor cells, and the cellular locations of these proteins include, but are not limited to, growth factors, growth factor receptors, cell signal receptors, cell to cell communication proteins, cellular structure proteins, transcription factors, nucleic acid processing proteins, DNA synthesis proteins, DNA repair proteins, cell division proteins, signal pathway proteins, metabolic pathway proteins, secreted proteins, cell membrane proteins, cytoplasmic proteins, nuclear proteins, membrane-bound proteins, and protein translation proteins. These proteins can be targets of therapeutic agents, predictors of patient outcome to specific therapeutic agents, and/or modulators of the cancer patient immune system.

The presently described SRM/MRM assays are useful for developing a personalized molecular profile of the tumor cells directly in the patient tumor tissue. Once the expression status of these proteins has been determined then specific therapeutic agents may be administered to the patient whereby such agents can interact with proteins detected and measured by the presently-described SRM/MRM assays, and either inhibit or enhance the function of those proteins to kill the tumor cells. In addition, therapeutic agents that induce the cancer patient's own immune system to kill the patient's own tumor cells can be administered to the cancer patient as part of this treatment regimen. Therapeutic agents that specifically target tumor-associated proteins and immune system-directed therapeutic agents may both be directly matched to the cancer patient molecular profile, as determined by the presently described SRM/MRM assays, providing for a personalized strategy for both targeted and immunologically based cancer treatment.

### DETAILED DESCRIPTION

Methods are provided for carrying out specific mass spectrometry-SRM/MRM assays useful for developing a molecular profile for a cancer patient, by precisely quantitating specific protease-digested peptides derived from a collection of proteins having a variety of functions and cellular locations in proteomic lysates prepared directly from patient tumor tissue. The process and assays can be used for understanding the molecular landscape of a patient's tumor and to guide selection of optimal cancer therapeutic agents that either directly kill the tumor cells or induce, initiate, support, and/or otherwise manipulate an active and successful immune response to the patient's own tumor cells, leading to improved patient survival. Cells from a biological sample of a cancer patient, such as, for example, formalin-fixed paraffin embedded (FFPE) tumor tissue, can be collected using, for example, the methodology of tissue microdissection. A lysate for mass spectrometry analysis can be prepared from the collected cells using, for example, the Liquid Tissue® reagents and protocol (see U.S. 7,473,532). The lysate can be analyzed using specific SRM/MRM assays as described in more detail below, where the assays are performed individually or in multiplex, and using protein detection/quantitation data from these SRM/MRM assays to develop a molecular profile for the patient/subject. These methods and the resulting SRM/MRM assay data can be used to determine an improved or optimal treatment regimen for the patient using therapeutic agents that directly function to inhibit protein function to kill tumor cells and inhibit their growth. In addition, the SRM/MRM assay data can be used to determine an improved or optimal treatment regimen for the patient using therapeutic agents that function to initiate, modulate, effect, enhance, and/or otherwise manipulate the cancer patient immune system to kill the tumor cells by directly interacting with one or more of the proteins detected and/or quantitated by the presently described SRM/MRM assays.

Determining a patient molecular profile by the described SRM/MRM assays may be performed on a variety of patient-derived samples including but not limited to blood, urine, sputum, pleural effusion, inflammatory fluid surrounding a tumor, normal tissue, and/or tumor tissue. In a particular embodiment, the sample is FFPE tissue, for example FFPE tumor tissue.

FFPE tissue is the most widely and advantageously available form of tissue, including tumor tissue, from cancer patients. Formaldehyde/formalin fixation of surgically removed tissue is by far the most common method of preserving cancer tissue samples worldwide and is the accepted convention in standard pathology practice. Aqueous solutions of formaldehyde are referred to as formalin. "100%" formalin consists of a saturated solution of formaldehyde (about 40% by volume or 37% by mass) in water, with a small amount of stabilizer, usually methanol, to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature. Molecular analytical methods that can analyze formalin fixed cancer tissue are the most accepted and heavily utilized methods for analysis of cancer patient tissue.

The most widely-used methodology presently applied to analyze protein expression in cancer patient tissue, especially FFPE tissue, is immunohistochemistry (IHC). IHC methodology uses an antibody to detect the protein of interest. The results of an IHC test are most often interpreted by a pathologist or histotechnologist. This interpretation is subjective and does not provide quantitative data that may be predictive of sensitivity to therapeutic agents that target specific proteins. In addition, studies involving IHC assays, such as the Her2 IHC test, suggest the results obtained from such tests may be wrong or misleading. This is likely because different laboratories use different rules for classifying positive and negative IHC status. Each pathologist running a test also may use different criteria to decide whether the results are positive or negative. In most cases, this happens when the test results are borderline, *i.e.* the results are neither strongly positive nor strongly negative. In other cases, cells present in one area of the cancer tissue section can test positive while cells in a different area of the cancer tissue section can test negative. Inaccurate IHC test results may mean that patients diagnosed with cancer do not receive the best possible care. If all or a specific region/cells of tumor tissue is truly positive for a specific protein but test results classify it as negative, physicians are unlikely to administer the correct therapeutic treatment to the patient. If tumor tissue is truly negative for expression of a specified protein but test results classify it as positive, physicians may use a specific therapeutic treatment even though the patient is not only unlikely to receive any benefit but also will be exposed to the agent's secondary risks. Accordingly, there is great clinical value in the ability to precisely detect and correctly evaluate quantitative levels of specific immune-based proteins in tumor tissue so that the patient will have the greatest chance of receiving a successful immunomodulatory treatment regimen while reducing unnecessary toxicity and other side effects.

Precise detection and correct evaluation of quantitative levels of specific proteins in tumor tissue may be effectively determined in a mass spectrometer by SRM/MRM methodology. This methodology detects and quantitates unique fragment peptides from specific proteins, including cancer biomarkers and immune-based proteins, in which the SRM/MRM signature chromatographic peak area of each peptide is determined within a complex peptide mixture present in a Liquid Tissue® lysate (see U.S. Pat. No. 7,473,532). One method of preparing a complex biomolecule sample directly from formalin-fixed tissue is described in U.S. Pat. No. 7,473,532, the contents of which are hereby incorporated by reference in their entirety. The methods described in U.S. Pat. No. 7,473,532 may conveniently be carried out using Liquid Tissue® reagents and protocol available from Expression Pathology Inc. (Rockville, Md.). For example, a composition comprising the formalin-fixed tumor sample and a reaction buffer can be heated at a temperature from 80°C to 100°C for a period of time from 10 minutes to 4 hours. Additionally, the resulting composition can be treated with an effective amount of a proteolytic enzyme selected from the group consisting of trypsin, chymotrypsin, and endoproteinase Lys-C for a period of time from 30 minutes to 24 hours at a temperature from 37°C to 65°C. In a particular embodiment, the proteolytic enzyme is trypsin. Quantitative levels of proteins can then be determined by the SRM/MRM methodology whereby the SRM/MRM signature chromatographic peak area of an individual specified peptide from each protein in a biological sample is compared to the SRM/MRM signature chromatographic peak area of a known amount of a "spiked" internal standard for each of the individual fragment peptides.

In one embodiment, the "spiked" internal standard is a synthetic version of the same exact protein-derived fragment peptide where the synthetic peptide contains one or more amino acid residues labeled with one or more heavy isotopes, such as ²H, ¹⁸O, ¹⁷O, ¹⁵N, ¹³C, or combinations thereof. Such isotope labeled internal standards are synthesized so that mass spectrometry analysis generates a predictable and consistent SRM/MRM signature chromatographic peak that is different and distinct from the native fragment peptide chromatographic signature peak and which can be used as comparator peak. Thus when the internal standard is "spiked" in known amounts into a protein or peptide preparation from a biological sample and analyzed by mass spectrometry, the SRM/MRM signature chromatographic peak area of the native peptide is compared to the SRM/MRM signature chromatographic peak area of the internal standard peptide, and this numerical comparison indicates either the absolute molarity and/or absolute weight of the native peptide present in the original proteomic preparation from the biological sample. Quantitative data for fragment peptides are displayed according to the amount of proteomic lysate analyzed per sample.

In order to develop and perform the SRM/MRM assay for a fragment peptide for a given protein, additional information beyond simply the peptide sequence may be utilized by the mass spectrometer. This additional information can be used to direct and instruct the mass spectrometer (e.g., a triple quadrupole mass spectrometer) to perform the correct and focused analysis of a specific fragment peptide. The additional information about a target peptide in general may include one or more of the mono isotopic mass of each peptide, its precursor charge state, the precursor m/z value, the m/z transition ions, and the ion type of each transition ion. An SRM/MRM assay may be effectively performed on a triple quadrupole mass spectrometer or an ion trap/quadrupole hybrid instrument. These types of mass spectrometers can analyze a single isolated target peptide within a very complex protein lysate containing hundreds of thousands to millions of individual peptides from all the proteins contained within a cell. This additional information provides the mass spectrometer with the correct directives to allow analysis of a single isolated target peptide within a very complex protein lysate. SRM/MRM assays also can be developed and performed on other types of mass spectrometer, including MALDI, ion trap, ion trap/quadrupole hybrid, or triple quadrupole instruments.

The foundation for a single SRM/MRM assay to detect and quantitate a specific protein in a biological sample is identification and analysis of one or more fragment peptides derived from the larger, full length version of the protein. This is because mass spectrometers are highly efficient, proficient, and reproducible instruments when analyzing very small molecules such as a single fragment peptide while mass spectrometers cannot efficiently, proficiently, or reproducibly detect and quantitate full length, intact proteins.

A candidate peptide for developing a single SRM/MRM assay for an individual protein may theoretically be any individual peptide that results from complete protease digestion, as for example digestion with trypsin, of the intact full length proteins. Surprisingly, however, many peptides are unsuitable for reliable detection and quantitation of any given protein - indeed, for some proteins no suitable peptide has yet been found. Accordingly, it is impossible to predict which is the most advantageous peptide to assay by SRM/MRM for a given protein, and therefore the specifically-defined assay characteristics about each peptide must be empirically discovered and determined. This is especially true when identifying the best SRM/MRM peptide for analysis in a protein lysate such as a Liquid Tissue® lysate from FFPE tissue. The presently described SRM/MRM assays designate one or more protease digested peptides (e.g. tryptic digested peptides) for each protein whereby each peptide has been discovered to be an advantageous peptide for SRM/MRM assay in Liquid Tissue® lysates prepared from formalin fixed patient tissue.

The presently described SRM/MRM assays detect and quantitate proteins that can be used to develop a molecular profile of the patient tumor tissue microenvironment. These proteins provide a wide variety of functions and are found in a wide variety of locations within the cell. These proteins include, but are not limited to growth factors, growth factor receptors, extracellular matrix proteins, nuclear transcription factors, epithelial cell differentiation factors, cell signaling proteins, immune cell differentiation factors, cell/cell recognition factors, self vs. tumor recognition factors, immune cell activation factors, immune cell inhibiting factors, and immune checkpoint proteins. Each of these individual proteins within this collection of proteins can be, and are, expressed by a wide variety of cells in the cancer patient including, but not limited to, all varieties of solid tissue cells such as epithelial tumor cells, normal epithelial cells, normal fibroblasts, tumor-associated fibroblasts, normal endothelial cells, tumor-associated endothelial cells, normal mesenchymal cells, and tumor-associated mesenchymal cells. Each of these proteins can be expressed by a wide variety of blood-born white blood cells including but not limited to all varieties of lymphocytes, such as B cells, T cells, macrophages, dendrites, mast cells, natural killer cells, eosinophils, neutrophils, and basophils. It is well known that in many cases each of these individual proteins can be expressed by both solid tissue cells and blood-born tissue cells.

The cellular expression patterns of each of these proteins can be very different depending on the health status of the individual. Under normal and usual healthy conditions these proteins maintain cellular health and a healthy immune system. With respect to maintaining a healthy immune system, cellular recognition of self is balanced with recognition of non-self, primarily through the major histocompatibility complex (MHC). The MHC is a set of cell surface proteins essential for the immune system to recognize foreign molecules in vertebrates, which in turn determines histocompatibility. The main function of MHC molecules is to bind to peptide fragments derived from pathogens and display them on the cell surface for recognition by the appropriate T-cells so an immune response can be mounted against the pathogen. MHC molecules mediate interactions of white blood cells which are immune cells, with other white blood cells or with solid tissue body cells. The MHC determines compatibility of donors for organ transplant, as well as one's susceptibility to an autoimmune disease via cross-reacting immunization. In humans, the MHC is also called the human leukocyte antigen (HLA). In a cell, protein molecules of the host's own phenotype or of other biologic entities are continually synthesized and degraded. Each MHC molecule on the cell surface displays a molecular fraction of a protein, called an epitope. The presented antigen can be either self or non-self. If the antigen is recognized as self then an organism's immune system is prevented from targeting its own cells with an immune killing response.

The MHC not only protects the body from pathogens but also plays a major role in the natural control of cancer cells. Cancer cells contain many mutated proteins and aberrantly expressed proteins that may be displayed by MHC molecules to alert the immune system. Tumor cells may also express normal proteins but in unusual locations, unusual ways, and/or in abnormal amounts providing signals to either mobilize or inhibit an immune response. A normal cell will display peptides from normal cellular protein turnover on its class I MHC, and immune system cells (white blood cells) will not be activated in response to them due to central and peripheral tolerance mechanisms that are mediated by specific proteins expressed normally on the surface of the white blood cells. When a cell expresses foreign proteins, such as after viral infection or in the case of aberrant protein expression by a cancer cell, a fraction of the class I MHC will display these peptides on the cell surface. Consequently, those white blood cells specific for the MHC :peptide complex will recognize and kill those cells, including cancer cells presenting the aberrant peptide. Alternatively, class I MHC itself can serve as an inhibitory ligand for that population of white blood cells, called natural killer cells (NKs), that kill virusinfected cells and cancer cells. Reduction in the normal levels of surface class I MHC, a mechanism employed by some viruses during immune evasion or in certain tumors, can help tumor cells to evade immune-mediated killing by inactivating NK-mediated cell killing.

A further example of cancer cells utilizing yet another strategy to evade immune surveillance is in the case of cancer cells aberrantly expressing the Programmed death-ligand 1 (PD-L1) checkpoint protein. PD-L1 is a transmembrane protein that plays a major role in suppressing the immune system during particular events such as pregnancy, tissue allografts, autoimmune disease and other disease states such as cancer. Normally the immune system reacts to foreign antigens where there is some accumulation in the lymph nodes or spleen which triggers a proliferation of antigen-specific CD8+ T cells that express PD-1. The binding of PD-L1 to PD-1 transmits an inhibitory signal which reduces the proliferation of these CD8+ T cells thereby signaling the immune system to disregard the cancer cells.

Upregulated aberrant expression of PD-L1 by cancer cells allows the cancer cells to evade the host immune system. An analysis of 196 tumor specimens from patients with renal cell carcinoma found that high tumor expression of PD-L1 was associated with increased tumor aggressiveness and a 4.5-fold increased risk of death. Ovarian cancer patients with higher expression of PD-L1 show a significantly poorer prognosis than those with lower expression. It is also known that PD-L1 expression correlates inversely with intraepithelial CD8+ T lymphocyte count, suggesting that PD-L1 on tumor cells may suppress antitumor CD8+ T cells. PD-L1 inhibitors are now either in use in routine cancer therapy or are in development as immuno-based cancer therapeutic agents. These agents show good response rates in many patients.

Immuno-based cancer therapy strategies are designed to initiate, modulate, strengthen or manipulate a patient's own immune system to fight and kill the patient's own cancer cells. Many forms of immunotherapy are being used for the treatment of cancer. The methods described herein provide quantitative protein expression data for proteins such as the PD-L1/PD-1 immune checkpoint proteins in patient tumor cells that may be used as part of potential treatment strategies with immune-based cancer therapeutic agents to initiate and/or modulate the balance of the tumor-activated immune response is the focus of the present description. An example of a single SRM/MRM assay for an immune checkpoint protein for the PD-L1 protein is described in the patent application PCT/US2015/010386, which is hereby incorporated by reference in its entirety.

The presently described SRM/MRM assays detect and quantitate expression of unique proteins expressed by many different cell types demonstrating many different functions and residing in many different locations within the cell. Each of the assays describes at least one optimal peptide that was found to be useful for reliable and reproducible detection and measurement of a single protein, where each assay can be performed individually or in multiplex with other peptides for other proteins. The protein and peptide listing is shown in Table 1. The proteins for which these assays have been developed are listed in Table 1 by one or more common and/or alternative names: ABCG2 (CDw338, cluster of differentiation w338), AIMP3 (ARS-interacting multi-functional proteins 3, p18), ALDH1B1 (ALDH5, ALDHX, aldehyde dehydrogenase 1 family member B1), alpha-Catenin, ARG2 (Arginase, type II), ARID1A (AT-rich interactive domain-containing protein 1A, B120, BAF250, BAF250a, BM029, C1orf4, ELD, MRD14, OSA1, P270, SMARCF1, hELD, hOSA1, CSS2), ARID2 (BAF200, p200, AT-rich interaction domain 2), ATM (ATM serine/threonine kinase, ATI, ATA, ATC, ATD, ATDC, ATE, TEL1, TELO1, ataxia-telangiectasia mutated), ATR (ATR serine/threonine kinase, FCTCS, FRP1, MEC1, SCKL, SCKL1), Bax (BCL2L4, BCL2 associated X protein, BCL2 associated X, apoptosis regulator), BCL2 (Bcl-2, PPP1R50, B-cell CLL/lymphoma 2, apoptosis regulator), Brachyury (T, SAVA, TFT brachyury transcription factor), BRCA1 (breast cancer 1, early onset, BRCC1, BROVCA1, IRIS, PNCA4, PPP1R53, PSCP, RNF53, FANCS, breast cancer 1, DNA repair associated), BRE (BRCC4, BRCC45, brain and reproductive organ-expressed [TNFRSF1A modulator]), CAD (CDG1Z, carbamoyl-phosphate synthetase 2, aspartate transcarbamylase, and dihydroorotase, GATD4, EIEE50), Cav-1 (BSCL3, CGL3, LCCNS, MSTP085, PPH3, VIP21, Caveolin 1), CD8b (CD8B1), CD16 (cluster of differentiation 16), CD20 (cluster of differentiation 20, MS4A1, B1, Bp35, CD20, CVID5, LEU-16, MS4A2, S7, membrane spanning 4-domains A1), CD99 (cluster of differentiation 99, HBA71, MIC2, MIC2X, MIC2Y, MSK5X), CD226 (cluster of differentiation 226, DNAM-1, DNAM1, PTA1, TLiSA1), CDK4 (CMM3, PSK-J3, cyclin-dependent kinase 4, cyclin dependent kinase 4), CDK6 (Cell division protein kinase 6, MCPH12, PLSTIRE, cyclin-dependent kinase 6), CDK10 (PISSLRE, cyclin-dependent kinase 10, cyclin dependent kinase 10), CDX2 (CDX-3, CDX2/AS, CDX3, caudal type homeobox 2, Cdx2), CHFR (RNF116, RNF196, checkpoint with forkhead and ring finger domains, E3 ubiquitin protein ligase, checkpoint with forkhead and ring finger domains), CHK1 (CHEK1, checkpoint kinase 1), c-JUN (JUN, AP-1, AP1, c-Jun, Jun proto-oncogene, AP-1 transcription factor subunit), CLK4 (CDC-like kinase 4, Dual specificity protein kinase CLK4), COX41 (Cytochrome c oxidase subunit 4 isoform 1, mitochondrial, Cytochrome c oxidase polypeptide IV, Cytochrome c oxidase subunit IV isoform 1, COX4, COX IV-1), COX5B (cytochrome c oxidase subunit Vb, COXVB, cytochrome c oxidase subunit 5B), COX6C (cytochrome c oxidase subunit 6C), DCTD (Deoxycytidylate deaminase, dCMP deaminase), DDR1 (CAK, CD167, DDR, EHGK2, MCK10, NEP, NTRK4, PTK3, PTK3A, RTK6, TRKE, discoidin domain receptor tyrosine kinase 1, CD167a, cluster of differentiation 167a), DGKA (Diacylglycerol kinase alpha, DAGK, DAGK1, DGK-alpha), Diablo (direct IAP binding protein with low pI, DFNA64, SMAC, Diablo, Diablo homolog, diablo IAP-binding mitochondrial protein), DICER1 (Endoribonuclease Dicer, Helicase with RNase motif, Helicase MOI, HERNA, KIAA0928), DUSP1 (Dual specificity protein phosphatase 1, CL100, HVH1, MKP-1, MKP1, PTPN10, dual specificity phosphatase 1), EIF3A (Eukaryotic translation initiation factor 3 subunit A EIF3, eIF3a, EIF3S10, P167, TIF32, eIF3-p170, eIF3-theta, p180, p185), EMSY (GL002, C11orf30, BRCA2 interacting transcriptional repressor), EPCAM (Epithelial cell adhesion molecule, DIAR5, EGP-2, EGP314, EGP40, ESA, HNPCC8, KS1/4, KSA, M4S1, MIC18, MK-1, TACSTD1, TROP1), ERCC2 (excision repair cross-complementation group 2, XPD, COFS2, EM9, TFIIH, TTD, XPD, TTD1, ERCC excision repair 2, TFIIH core complex helicase subunit), FOXA1 (hepatocyte nuclear factor 3-alpha, HNF3A, TCF3A, forkhead box A1), FOXC1 (Forkhead box C1, ARA, FKHL7, FREAC-3, FREAC3, IGDA, IHG1, IRID1, RIEG3, ASGD3), FOXC2 (Forkhead box protein C2, forkhead-related protein, FKHL14, transcription factor FKH-14, mesenchyme fork head protein 1, MFH1), FOXF2 (Forkhead box protein F2, Forkhead-related activator 2, FREAC-2, Forkhead-related protein FKHL6, Forkhead-related transcription factor 2), gamma-catenin (Plakoglobin, JUP, ARVD12, CTNNG, DP3, DPIII, PDGB, PKGB, junction plakoglobin), GDA (guanine deaminase, guanase, guanine aminase, guanine aminohydrolase, GAH), GJA1 (Gap junction alpha-1 protein, connexin 43, Cx43, AVSD3, CMDR, CX43, EKVP, GJAL, HLHS1, HSS, ODDD, PPKCA), GLS (glutaminase kidney isoform-mitochondrial, K-glutaminase, L-glutamine amidohydrolase), GLUT1 (glucose transporter 1, solute carrier family 2 facilitated glucose transporter member 1, SLC2A1, CSE, DYT17, DYT18, DYT9, EIG12, GLUT, GLUT-1, GLUT1, GLUT1DS, HTLVR, PED, SDCHCN, solute carrier family 2 member 1), GSTM1 (Glutathione S-transferase Mu 1, GST1, GSTM1-1, GSTM1a-1a, GSTM1b-1b, GTH4, GTM1, H-B, MU, MU-1), GSTM3 (Glutathione S-transferase M3, GST5, GSTB, GSTM3-3, GTM3), GSTP1 (glutathione s-transferase P, DFN7, FAEES3, GST3, GSTP, HEL-S-22, PI, glutathione S-transferase pi 1), GSTT1 (glutathione S-transferase theta-1), HAI2 (SPINT2, DIAR3, HAI-2, Kop, PB, serine peptidase inhibitor, Kunitz type, 2, serine peptidase inhibitor, Kunitz type 2), hCNT3 (concentrative Na(+)-nucleoside cotransporter, CNT3, SLC28A3) HDAC6 (Histone deacetylase 6, CPBHM, HD6, PPP1R90, JM21), HK2 (hexokinase 2, HKII, HXK2), HLA-A (Aw-33, Aw-74, major histocompatibility complex, class I, A, HLA-A11, HLA-A33, HLADQB1, HLA-DRB1) HLA-B (AS, SPDA1, HLA-B, Bw-47, Bw-50, major histocompatibility complex, class I, B, B-4901, B-5001, HEL-S-83, HLA-B*45ZJ, HLA-B-3506, HLA-B-3905, HLA-B-5502, HLA-B-5602, HLA-B15, HLA-B39, HLA-B49, HLA-B50, HLA-B55, HLA-B59, HLA-B61, HLA-Cw, HLA-DRB1), HLA-DMA (D6S222E, DMA, HLADM, RING6, HLA-DMA, major histocompatibility complex, class II, DM alpha), HLA-DQA1 (HLA class II histocompatibility antigen, DQ alpha 1 chain, DC-1 alpha chain, DC-alpha, HLA-DCA, MHC class II DQA1), HLA-DRA (HLA-DRA1, MLRW, major histocompatibility complex, class II, DR alpha), HPV16E6 (Human papillomavirus type 16 E6), HR23B (RAD23B, HHR23B, P58, RAD23 homolog B, nucleotide excision repair protein), IDH1 (HEL-216, HEL-S-26, IDCD, IDH, IDP, IDPC, PICD, isocitrate dehydrogenase (NADP(+)) 1, cytosolic), IDH2 (D2HGA2, ICD-M, IDH, IDHM, IDP, IDPM, mNADP-IDH, isocitrate dehydrogenase (NADP(+)) 2, mitochondrial), IGF2 (C11orf43, GRDF, IGF-II, PP9974, insulin like growth factor 2), IGF2BP3 (CT98, IMP-3, IMP3, KOC, KOC1, VICKZ3, insulin like growth factor 2 mRNA binding protein 3), IKZF1 (DNA-binding protein Ikaros, Hs.54452, IK1, IKAROS, LYF1, LyF-1, PPP1R92, PRO0758, ZNFN1A1, CVID13, IKAROS family zinc finger 1), IKZF3 (AIO, AIOLOS, ZNFN1A3, IKAROS family zinc finger 3), IL13RA2 (CD213A2, CT19, IL-13R, IL13BP, interleukin 13 receptor subunit alpha 2), IL-15 (IL15, interleukin 15), IL-8 (IL8, CXCL8, chemokine (C-X-C motif) ligand 8, GCP-1, GCP1, LECT, LUCT, LYNAP, MDNCF, MONAP, NAF, NAP-1, NAP1, IL8, C-X-C motif chemokine ligand 8, Interleukin-8), IRS2 (IRS-2, insulin receptor substrate 2), LAG3 (cluster of differentiation 223, CD223, lymphocyte activating 3), LGR5 (FEX, GPR49, GPR67, GRP49, HG38, leucine-rich repeat containing G protein-coupled receptor 5, leucine rich repeat containing G protein-coupled receptor 5), LIG4 20 (LIG4S, DNA ligase 4), MCT1 (monocarboxylate transporter 1, SLC16A1, HHF7, MCT, MCT1D, solute carrier family 16 member 1), MENA (ENAH, ENA, NDPP1, enabled homolog (Drosophila), actin regulator), MENAINV, MFF (mitochondrial fission factor, C2orf33), MIG6 (mitogen-inducible gene 6 protein, ERBB receptor feedback inhibitor 1, ERRFI1), MLH1 (MutL homolog 1, colon cancer, nonpolyposis type 2, mutL homolog 1, COCA2, FCC2, HNPCC, HNPCC2, hMLH1), MMP7 (matrilysin, MMP-7, MPSL1, PUMP-1, matrix metallopeptidase 7), MMP9 (CLG4B, GELB, MANDP2, MMP-9, 92 kDa type IV collagenase, 92 kDa gelatinase, gelatinase B, matrix metallopeptidase 9), MRE11 (ATLD, HNGS1, MRE11B, MRE11A, MRE11 homolog A, double strand break repair nuclease, MRE11 homolog, double strand break repair nuclease), MSH2 (mutS homolog 2, COCA1, FCC1, HNPCC, HNPCC1, LCFS2), MSH6 (mutS homolog 6, GTBP, GTMBP, HNPCC5, HSAP, p160), MUC4 (ASGP, HSA276359, MUC-4, mucin 4, cell surface associated), ND1 (MTMT-NADH dehydrogenase, subunit 1 [complex I]), NEDD8 (NEDD-8, neural precursor cell expressed, developmentally downregulated 8), Nibrin (NBN, AT-V1, AT-V2, ATV, NBS, NBS1, P95, nibrin), NKp30 (natural cytotoxicity triggering receptor 3, CD337, cluster of differentiation 337, NCR3, 1C7, CD337, LY117, MALS), NKp44 (natural cytotoxicity triggering receptor 2, CD336, cluster of differentiation 336, NCR2, CD336, LY95, NK-p44, NKP44), NKp80 (killer cell lectin-like receptor subfamily F member 1, Lectin-like receptor F1, C-type lectin domain family 5 member C, KLRF1), NNMT (nicotinamide N-methyltransferase), NT5C (5', 3'-nucleotidase, cytosolic, 5'(3')-deoxyribonucleotidase, cytosolic type, deoxy-5'-nucleotidase 1, DNT, DNT1, HEL74, P5N2, PN-I, PN-II, UMPH2, cdN, dNT-1), NT5C2 (cytosolic purine 5'-nucleotidase, Cytosolic 5'-nucleotidase II, cytosolic 5'-nucleotidase 3A, pyrimidine 5'-nucleotidase), NT5C3A (cytosolic 5'-nucleotidase 3, cytosolic 5'-nucleotidase 3A, pyrimidine 5'-nucleotidase, p56, NT5C3, P5'N-1, P5N-1, PN-I, POMP, PSN1, UMPH, UMPH1, cN-III, hUMP1, p36), P21 (cyclin-dependent kinase inhibitor 1 or CDK-interacting protein 1, CDKN1A, CAP20, CDKN1, CIP1, MDA-6, SDI1, WAF1, p21CIP1, cyclin-dependent kinase inhibitor 1A, cyclin dependent kinase inhibitor 1A), P27 (cyclin-dependent kinase inhibitor 1B, CDKN1B, CDKN4, KIP1, MEN1B, MEN4, P27KIP1, cyclin dependent kinase inhibitor 1B), PARP1 (poly [ADP-ribose] polymerase 1, NAD+ ADP-ribosyltransferase 1, poly[ADP-ribose] synthase 1, ADPRT, ADPRT 1, ADPRT1, ARTD1, PARP, PARP-1, PPOL, pADPRT-1), PARP16 (poly (ADP-ribose) polymerase family member 16, ARTD15, C15orf30, pART15), PARP4 (ADPRTL1, ARTD4, PARP-4, PARPL, PH5P, VAULT3, VPARP, VWA5C, p193, poly(ADP-ribose) polymerase family member 4), Paxillin (PXN), PDK1 (PDPK1, PDPK2, PRO0461, PDPK2P, Phosphoinositide-dependent kinase-1, 3-phosphoinositide dependent protein kinase 1), PD-L2 (PD-1-ligand 2), PFKFB3 (IPFK2, PFK2, iPFK-2, 6-phosphofructo-2-kinase/fructose-2,6-biphosphatase 3), PI3KCA (phosphatidylinositol-4,5-bisphosphate 3-kinase, catalytic subunit alpha, CLOVE, CWS5, MCAP, MCM, MCMTC, PI3K, p110-alpha, PI3K-alpha), PIM1 (Proto-oncogene serine/threonine-protein kinase Pim-1, PIM, Pim-1 proto-oncogene, serine/threonine kinase), PIM2 (Serine/threonine-protein kinase Pim-2, Pim-2 proto-oncogene, serine/threonine kinase), PKM (Pyruvate kinase isozymes M1/M2 [PKM1/M2], also known as pyruvate kinase muscle isozyme [PKM], pyruvate kinase type K, cytosolic thyroid hormone-binding protein [CTHBP], thyroid hormone-binding protein 1 [THBP1], opa-interacting protein 3 [OIP3], CTHBP, HEL-S30, OIP3, PK3, PKM2, TCB, THBP1, pyruvate kinase, muscle), PMS2 (HNPCC4, PMS2CL, PMSL2, MLH4, PMS1 homolog 2, mismatch repair system component), PSMA (FOLH1, FGCP, FOLH, GCP2, GCPII, NAALAD1, NAALAdase, PSM, mGCP, folate hydrolase [prostate-specific membrane antigen] 1, folate hydrolase 1), PRKDC (DNA-PKcs, DNAPK, DNPK1, HYRC, HYRC1, XRCC7, p350, IMD26, protein kinase, DNA-activated, catalytic polypeptide), QPRT (Nicotinate-nucleotide pyrophosphorylase [carboxylating], Quinolinate phosphoribosyltransferase [decarboxylating], QAPRTase, QPRTase), RAD50 (NBSLD, RAD502, hRad50, RAD50 double strand break repair protein), SAMHD1 (SAM domain and HD domain-containing protein 1, CHBL2, DCIP, HDDC1, MOP-5, SBBI88, SAM and HD domain containing deoxynucleoside triphosphate triphosphohydrolase 1), SIAH2 (hSiah2, siah E3 ubiquitin protein ligase), SIRT1 (SIR2L1, SIR2, hSIR2, SIR2alpha, Sirtuin 1, NAD-dependent deacetylase sirtuin-1), SLAMF6 (CD352, KALI, KALIb, Ly108, NTB-A, NTBA, SF2000, SLAM family member 6), SLC34A2 (Sodium-dependent phosphate transport protein 2B [NaPi2b], NAPI-3B, NAPI-IIb, NPTIIb, solute carrier family 34 member 2), SLC46A1 (Solute carrier family 46 [folate transporter], member 1 [SLC46A1] also known as proton-coupled folate 15 transporter [PCFT], G21, HCP1, PCFT, solute carrier family 46 member 1), SMARCA4 (transcription activator BRG1, ATP-dependent helicase SMARCA4, BAF190, BAF190A, BRG1, MRD16, RTPS2, SNF2, SNF2L4, SNF2LB, SWI2, hSNF2b, CSS4, SWI/SNF related, matrix associated, actin dependent regulator of chromatin, subfamily a, member 4), SMO (FZD11, Gx, SMOH, smoothened, frizzled class receptor, CRJS), SOD1 (ALS, ALS1, HEL-S-44, IPOA, SOD, hSod1, homodimer, superoxide dismutase 1, soluble, superoxide dismutase 1), SOD2 (IPOB, MNSOD, MVCD6, IPO-B, Mn-SOD, superoxide dismutase 2, mitochondrial, superoxide dismutase 2), SOX9 (transcription factor SOX-9, CMD1, CMPD1, SRA1, SRXX2, SRXY10, SRY-box 9), SPRY2 (Sprouty homolog 2, hIGAN3, sprouty RTK signaling antagonist 2), TBX21 (T-box transcription factor TBX21, T-PET, T-bet, TBET, TBLYM, T-box 21), TJP1 (zonula occludens-1 ZO-1, tight junction protein-1, ZO-1, Tight junction protein 1), TMSB15A (thymosin beta-15A, TMSB15, TMSB15B, TMSL8, TMSNB, Tb15, TbNB, thymosin beta 15a), TPX2 (Targeting protein for Xklp2, C20orf1, C20orf2, DIL-2, DIL2, FLS353, GD:C20orf1, HCA519, HCTP4, REPP86, p100, microtubule nucleation factor), TRIM24 (tripartite motifcontaining 24, transcriptional intermediary factor 1α, PTC6, RNF82, TF1A, TIF1, TIF1A, TIF1ALPHA, hTIF1, tripartite motif containing 24), TRKA (tropomyosin receptor kinase A (TrkA), high affinity nerve growth factor receptor, neurotrophic tyrosine kinase receptor type 1, TRK1-transforming tyrosine kinase protein, MTC, TRK, TRK1, Trk-A, p140-TrkA, neurotrophic receptor tyrosine kinase 1), WT-1 (Wilms tumor protein, AEWS-GUD, NPHS4, WAGR, WIT-2, WT33, Wilms tumor 1), XPO1 (exportin 1, chromosomal maintenance 1, CRM1, emb, exp1), XRCC4 (DNA repair protein XRCC4, X-ray repair cross-complementing protein 4, SSMED, X-ray repair complementing defective repair in Chinese hamster cells 4, Xray repair cross complementing 4), XRCC5 (KARP-1, KARP1, KU80, KUB2, Ku86, NFIV, Ku80, X-ray repair complementing defective repair in Chinese hamster cells 5, X-ray repair cross complementing 5), XRCC6 (X-ray repair complementing defective repair in Chinese hamster cells 6, CTC75, CTCBF, G22P1, KU70, ML8, TLAA, X-ray repair cross complementing 6), AMD1 (Adenosylmethionine Decarboxylase 1), FCER1G (Fc fragment of IgE receptor Ig), RAD51C (BROVCA3), PML (TRIM19, Promyelocytic leukemia protein), IL12RB2 (Interleukin 12 receptor), SPAG5 (Sperm Associated Antigen 5), NRAS (Neuroblastoma RAS Viral (V-Ras) Oncogene Homolog), HRAS (Harvey rat sarcoma viral oncogene homolog), MLH3 (DNA Mismatch Repair Protein Mlh3), CEACAM6 (carcinoembryonic antigen related cell adhesion molecule 6), LGALS9 (Galectin 9), RPA2 (replication protein A2), RPA1 (Replication protein A1), FEN1 (Flap Structure-Specific Endonuclease 1), LIG3 (DNA Ligase 3), BLM (Bloom syndrome RecQ like helicase), B2M (beta-2-microglobulin), RNPEP (Arginine Aminopeptidase), IL12A (Interleukin 12A), IL12B (Interleukin 12B), YES1 (V-Yes-1 Yamaguchi Sarcoma Viral Oncogene Homolog 1), NTRK1 (Aliases for NTRK1 Gene Neurotrophic Receptor Tyrosine Kinase 1), NTRK2 (Aliases for NTRK1 Gene Neurotrophic Receptor Tyrosine Kinase 2), NTRK3 (Aliases for NTRK1 Gene Neurotrophic Receptor Tyrosine Kinase 3), and RB1 (retinoblastoma 1).

Surprisingly, it was found that many potential peptide sequences derived from cleavage of the proteins listed in Table 1 are unsuitable or ineffective for use in mass spectrometry-based SRM/MRM assays for reasons that are not immediately evident. As it was not possible to predict the most suitable peptides for MRM/SRM assay, it was necessary to experimentally identify modified and unmodified peptides in actual Liquid Tissue lysates to develop a reliable and accurate SRM/MRM assay for each designated protein. While not wishing to be bound by any theory, it is believed that some peptides might, for example, be difficult to detect by mass spectrometry because they do not ionize well or produce fragments distinct from other proteins. Peptides may also fail to resolve well in chromatography separation or may adhere to glass or plastic ware.

The peptides found in Table 1 were derived from their respective designated proteins by protease digestion of all the proteins within a complex Liquid Tissue® lysate prepared from cells procured from human formalin fixed cancer tissue. Unless noted otherwise, in each instance the protease was trypsin. The Liquid Tissue® lysate was then analyzed by mass spectrometry to determine those peptides derived from a designated protein that are detected and analyzed by mass spectrometry. Identification of a specific preferred subset of peptides for mass spectrometric analysis is based on discovery under experimental conditions of which peptide or peptides from a protein ionize in mass spectrometry analyses of Liquid Tissue® lysates, and thus demonstrate the ability of the peptide to result from the protocol and experimental conditions used in preparing a Liquid Tissue® lysate to be analyzed by the methodology of mass spectrometry.

The optimal peptides suitable for detecting this collection of proteins were discovered as follows. Protein lysates from cells procured directly from formalin (formaldehyde) fixed tissue were prepared using the Liquid Tissue® reagents and protocol, involving collecting cells into a sample tube via tissue microdissection, followed by heating the cells in the Liquid Tissue® buffer for an extended period of time. Once the formalin-induced cross linking has been negatively affected, the tissue/cells are then digested to completion in a predictable manner using a protease, such as, for example, the protease trypsin. Each protein lysate is turned into a collection of peptides by digestion of intact polypeptides with the protease. Each Liquid Tissue® lysate was analyzed (e.g., by ion trap mass spectrometry) to perform multiple global proteomic surveys of the peptides where the data was presented as identification of as many peptides as could be identified by mass spectrometry from all cellular proteins present in each protein lysate. An ion trap mass spectrometer or another form of a mass spectrometer that is capable of performing global profiling for identification of as many peptides as possible from a single complex protein/peptide lysate is typically employed. Ion trap mass spectrometers however may be the best type of mass spectrometer for conducting global profiling of peptides.

Although an SRM/MRM assay can be developed and performed on any type of mass spectrometer, including a MALDI, ion trap, ion trap/quadrupole hybrid, or triple quadrupole, the most advantageous instrument platform for an SRM/MRM assay is often considered to be a triple quadrupole instrument platform. Once as many peptides as possible were identified in a single MS analysis of a single lysate under the conditions employed, then that list of peptides was collated and used to determine the proteins that were detected in that lysate. That process was repeated for multiple Liquid Tissue® lysates, and the very large list of peptides was collated into a single dataset. That type of dataset can be considered to represent the peptides that can be detected by mass spectrometry in the type of biological sample that was analyzed (after protease digestion), and specifically in a Liquid Tissue® lysate of the biological sample, and thus includes the peptides for each of the designated proteins.

In one embodiment, the tryptic peptides identified as useful in the determination of absolute or relative amounts of the designated proteins are listed in Table 1 below. Each of these peptides was detected by mass spectrometry in Liquid Tissue® lysates prepared from FFPE tissue. Thus, each peptide can be used to develop a quantitative SRM/MRM assay for a designated protein in human biological samples, including directly in formalin-fixed patient tissue.

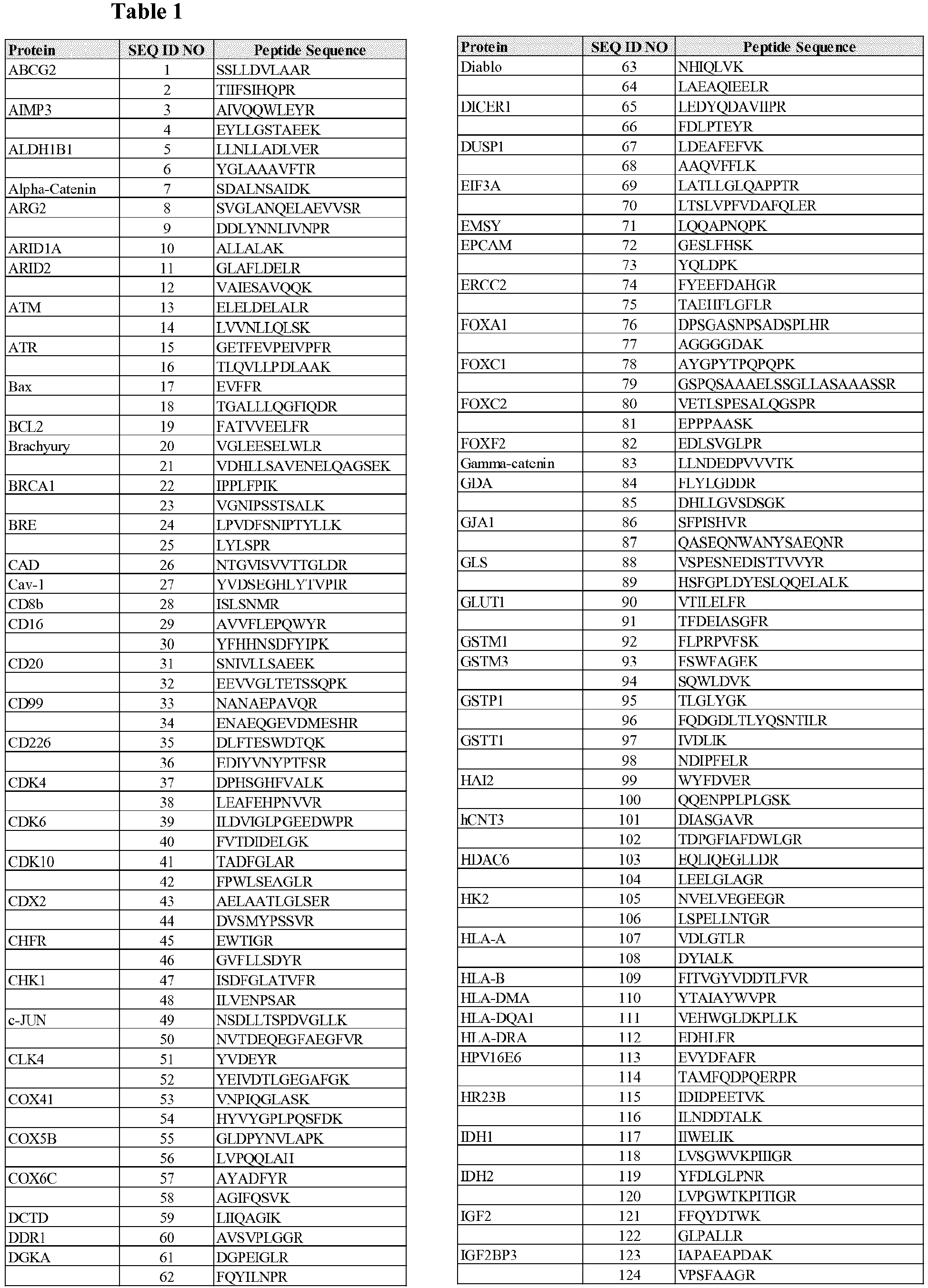

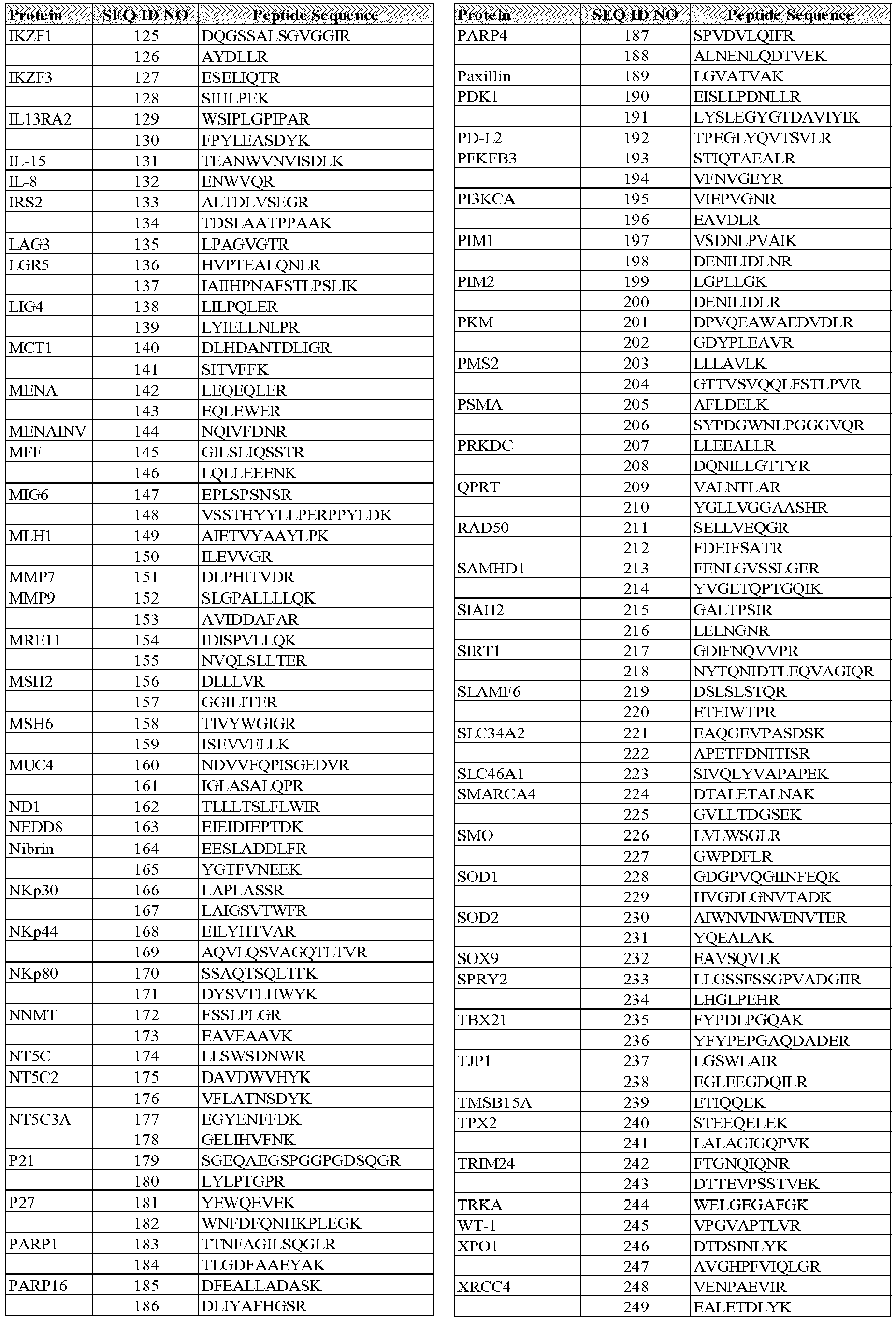

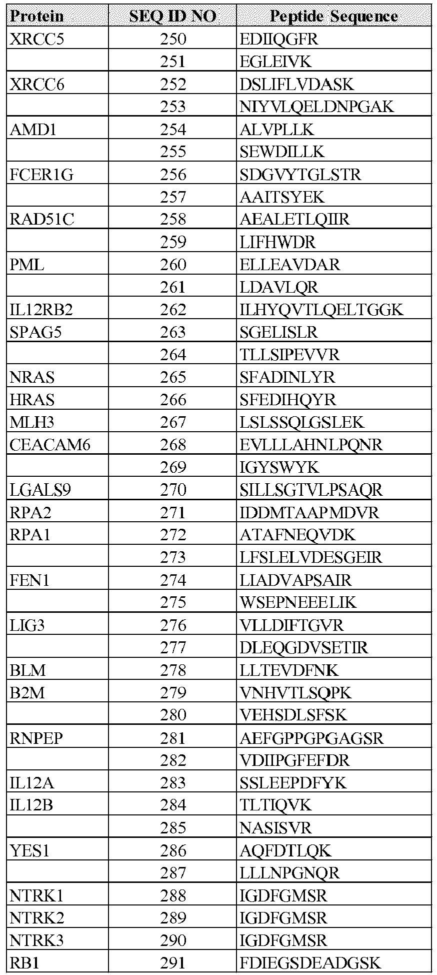

Specific and unique characteristics about specific fragment peptides from each designated protein were developed by analysis of all fragment peptides on both an ion trap and triple quadrupole mass spectrometers. That information must be determined experimentally for each and every candidate SRM/MRM peptide directly in Liquid Tissue® lysates from formalin fixed samples/tissue; because, interestingly, not all peptides from any designated protein can be detected in such lysates using SRM/MRM as described herein. Fragment peptides that cannot detected are not candidate peptides for developing an SRM/MRM assay for use in quantitating peptides/proteins directly in Liquid Tissue® lysates from formalin fixed samples/tissue.

In some embodiments, one or more fragment peptides detected and/or quantitated in an SRM assay described herein correspond to SEQ ID NO: 1-291 in Table 1 above.

In some embodiments, multiplex detection and/or quantitation of two or more, three or more, five or more, 6 or more, 7 or more, 8 or more, 9 or more, 10 or more, 15 or more, 20 or more, etc...is performed from the fragment peptides selected from SEQ ID NO: 1-291.

In some embodiments, a SRM/MRM assay for a specific fragment peptide is performed on a triple quadrupole mass spectrometer. An experimental sample analyzed by a particular protein SRM/MRM assay is, for example, a Liquid Tissue® protein lysate prepared from a tissue that had been formalin fixed and paraffin embedded. Data from such an assay can indicate the presence of the unique SRM/MRM signature peak for this fragment peptide in the formalin fixed sample.

Specific transition ion characteristics for a given peptide is used to not simply detect a particular fragment peptide but to quantitatively measure a particular fragment peptide in formalin fixed biological samples. These data indicate absolute amounts of this fragment peptide as a function of the molar amount of the peptide per microgram of protein lysate analyzed. Assessment of corresponding protein levels in tissues based on analysis of formalin fixed patient-derived tissue can provide diagnostic, prognostic, and therapeutically-relevant information about each particular patient. In one embodiment, methods are provided for measuring the level of each of the proteins listed in Table 1 in a biological sample, comprising detecting and/or quantifying the amount of one or more fragment peptides in a protein digest prepared from said biological sample using mass spectrometry; and calculating the level of modified or unmodified protein in said sample; where the level is a relative level or an absolute level. Quantifying one or more modified or unmodified fragment peptides may be achieved by determining the amount of each of the fragment peptides in a biological sample by comparison to an added internal standard peptide of known amount, where each of the fragment peptides in the biological sample is compared to an internal standard peptide having the same amino acid sequence. The internal standard may be an isotopically labeled internal standard peptide containing, for example, one or more heavy stable isotopes such as ¹⁸O, ¹⁷O, ³⁴S, ¹⁵N, ¹³C, ²H or combinations thereof. One molecule of peptide is derived from a single molecule of protein and therefore a measure of the molar amount of the peptide provides a direct measurement of the molar amount of the protein from which the peptide was derived.

The method for measuring the level of a designated protein in a biological sample described herein (or fragment peptides as surrogates thereof) may be used as a diagnostic indicator of cancer in a patient or subject. The results from measurements of the level of a designated protein may be employed to determine the diagnostic stage/grade/status of a cancer by correlating (e.g., comparing) the level of the protein found in a tissue with the level of that protein found in normal and/or cancerous or precancerous tissues. The results from measurements of the level of a designated protein also may be employed to determine which cancer therapeutic agents to treat a cancer patient with and thus the most optimal cancer treatment regimen.

The tissue protein expression landscape is highly complex whereby multiple proteins expressed by multiple types of solid tissue cells and localized/non-localized immune cells require multiple assays for multiple therapeutic agent indications. This level of protein assay complication can be analyzed by the presently described SRM/MRM assays. These assays are designed to substantially simultaneously (or at substantially the same time or substantially together) detect and quantitate many different proteins having a variety of molecular functions, where the proteins include, but are not limited to soluble proteins, membrane-bound proteins, nuclear factors, differentiation factors, proteins that modulate cell-to-cell interactions, secreted proteins, immune checkpoint proteins, growth factors, growth factor receptors, cell signaling proteins, immune inhibitory proteins, cytokines, and lymphocyte-activating/inhibiting factors.

Tissue microdissection can advantageously be used to procure pure populations of tumor cells from patient tumor tissue for protein expression analysis using the SRM/MRM assays in order to determine the molecular profile that specifically defines tumor cell status for the patient. Tissue microdissection of tumor tissue can be performed using the process of laser induced forward transfer of cells and cell populations utilizing DIRECTOR® technology.

Methods for using a DIRECTOR® slide for laser induced forward transfer of tissue via utilization of an energy transfer interlayer coating are described in U.S. Pat. No. 7,381,440, the contents of which are hereby incorporated by reference in their entirety. However, microdissecting pure populations of tumor cells may likely ignore the protein expression signature/profile of the cells expected to kill the tumor cells, i.e. tumor infiltrating lymphocytes (TILs). This limitation can be overcome by utilizing tissue microdissection to procure, in addition to a pure population of tumor cells, a pure population of TILs whereby areas of the tissue containing large populations of TILs can be collected and processed for protein expression analysis using the described SRM/MRM assays. Through collection and analysis of two distinct cell populations, the patient immune profile can be determined to inform the most optimal treatment regimen for the patient whereby the immune system can be modulated by specific immune-modulating agents for optimal immune-mediated tumor cell killing and the tumor cells can be targeted for killing by targeted therapeutic agents and/or immune-mediated tumor cell killing.

While tissue microdissection produces pure populations of specified cell populations from patient tissue for SRM/MRM analysis, the majority of tumor tissues do not show suitably large areas of distinct populations of TILs to be microdissected. In most cases, TILs are sparsely interspersed amongst the heterogeneous complex tissue microenvironment so that relatively pure populations of tumor cells can be effectively analyzed but analysis of pure populations of TILs is not routinely effective. Tumor tissue-derived TILs express many proteins important to informing the positive manipulation of the immune response using immune system modulatory agents and thus should be analyzed. This limitation can be overcome by preparing an analyzable protein lysate for the described SRM/MRM assays from the entire area of the tumor microenvironment present within the patient tissue whereby the lysate comprises a proteomic representation of the entire complex milieu of many different cell types including but not limited to tumor cells, benign non-tumor cells, and immune cells. In this way a highly complex patient-specific molecular profile can be determined capturing the entire molecular landscape of the patient tumor environment. In addition, analysis of purified populations of tumor cells as collected by tissue microdissection of a serial section from the same tissue can be compared and contrasted to the overall tumor microenvironment landscape. This approach functionally separates the tumor cell profile from the immune cell profile to identify immune response proteins most likely expressed by localized TILs and/or immune cells not present in the tissue sample, and the effect those proteins may have on the tumor immune landscape.

The presently described SRM/MRM assays detect and quantitate expression of specific proteins in lysates prepared from solid tumor tissue; however, unless pure populations of cells are collected and analyzed these assays cannot provide detailed information about which cells express which proteins. This is important because aberrant protein expression is common in the tumor microenvironment, as for example when tumor cells express immune inhibitory factors that are usually expressed solely by normal cells, normal lymphocytic cells, and/or TILs. Thus, when expression of candidate therapeutic protein targets has been detected and quantitated by the described SRM/MRM assays a follow-up assay may be necessary to provide the missing cellular localization information. The method to achieve cellular expression context is immunohistochemistry. Understanding which proteins are expressed within the tumor microenvironment and which cells express these proteins may advantageously inform optimal treatment decisions to modulate the patient's own immune response to seek out and kill the tumor cells. The presently described SRM/MRM assays and analysis process provide the ability to detect and quantitate protein targets of immunomodulatory cancer therapeutic agents directly in patient tumor tissue.

An advantageous approach for tumor cell killing is to use a combination therapy whereby immunomodulatory agents are used in combination with tumor cell targeting agents synergistically for optimal patient response. SRM/MRM assays can be used to determine the quantitative expression status in patient tumor tissue of oncoprotein targets for which inhibitory therapeutic agents have been developed. Examples of SRM/MRM assays to determine the quantitative status of oncoproteins are described, for example, for the Met protein (see US Patent 9,372,195) and the IGF-1R protein (see US Patent 8,728,753). The drugs crizotinib and cabozantinib inhibit Met protein function while figitumumab and cixutumumab inhibit IGF-1R protein function. The information from these assays can be combined with information from the presently described SRM/MRM assays to understand the immune status of the tumor tissue. Together, both datasets may be used to inform a treatment regimen for a targeted and immunebased combinatorial therapeutic approach. As for example, if a patient's tumor cells were determined by the SRM/MRM methodology to overexpress both the PD-L1 protein and the Met protein then a logical combinatorial treatment regimen might include administering nivolumab (PD-1 inhibitor) or atezolizumab (PD-L1 inhibitor) in combination with crizotinib (Met inhibitor). The result of such an approach would be to optimally utilize therapeutic agents that specifically target and kill the tumor cells along with arming the patient immune system to attack and kill the tumor cells.

Thus, in some embodiments, combining multiplex detecting and quantitating of two or more fragment peptides corresponding to SEQ ID NO: 1-291 with analysis of other oncoproteins that drive growth of the patient tumor cells can be advantageous. This can allow a targeted cancer therapeutic agent that inhibits or modulates the function of the oncoprotein to inhibit growth of the patient tumor cells to be administered to the patient in combination with an immunomodulatory cancer therapeutic agent that interacts with one or more of the proteins to initiate, enhance, manipulate, and/or otherwise modulate the cancer patient immune response to attack and kill the patient tumor cells.

Because both nucleic acids and protein can be analyzed from the same Liquid Tissue® biomolecular preparation it is possible to generate additional information about drug treatment decisions from the nucleic acids in the same sample analyzed with the presently described SRM/MRM assays. A specific protein can be found by the presently described SRM/MRM assays to be expressed by certain cells at increased levels while at the same time information about the mutation status of specific genes and/or the nucleic acids and proteins they encode (e.g., mRNA molecules and their expression levels or splice variations) can be obtained. Those nucleic acids can be examined, for example, by one or more, two or more, or three or more of: sequencing methods, polymerase chain reaction methods, restriction fragment polymorphism analysis, identification of deletions, insertions, and/or determinations of the presence of mutations, including but not limited to, single base pair polymorphisms, transitions, transversions, or combinations thereof.

Some methods are further comprising administering to a patient or subject from which the biological sample was obtained a therapeutically effective amount of a cancer therapeutic agent,
wherein the cancer therapeutic agent and/or amount of the cancer therapeutic agent administered is based upon detection of and/or amount of the one or more fragment peptides selected from SEQ ID NO: 1-291, preferably multiplex detection of and/or amount of two or more fragment peptides selected from SEQ ID NO: 1-291, and
wherein the cancer therapeutic agent is a targeted agent that interacts with one or more proteins that correspond to the one or more fragment peptides selected from SEQ ID NO: 1-291.

Some methods are further comprising administering to a patient or subject from which the biological sample was obtained a therapeutically effective amount of a cancer therapeutic agent,
wherein the cancer therapeutic agent and/or amount of the cancer therapeutic agent administered is based upon detection of and/or amount of the one or more fragment peptides selected from SEQ ID NO: 1-291, preferably multiplex detection of and/or amount of two or more fragment peptides selected from SEQ ID NO: 1-291, and
wherein the cancer therapeutic agent is an immunomodulatory cancer therapeutic agent whose function is to initiate, enhance, manipulate, and/or otherwise modulate the cancer patient immune response to attack and kill said patient tumor cells.

Some methods are further comprising combining multiplex detecting and quantitating two or more fragment peptides corresponding to SEQ ID NO: 1-291 with analysis of other oncoproteins that drive growth of the patient tumor cells, wherein a targeted cancer therapeutic agent that inhibits or modulates the function of the oncoprotein to inhibit growth of the patient tumor cells is administered to the patient in combination with an immunomodulatory cancer therapeutic agent that interacts with one or more of the proteins to initiate, enhance, manipulate, and/or otherwise modulate the cancer patient immune response to attack and kill the patient tumor cells.

## Claims

1. A method of developing a protein expression profile in a biological sample of formalin fixed tumor tissue obtained from a cancer patient, the method comprising
detecting and quantifying a level of one or more fragment peptides in a protein digest, preferably a trypsin digest, prepared from the biological sample of formalin fixed tumor tissue, preferably primary or secondary tumor tissue, using mass spectrometry; and
calculating a level of a corresponding protein or proteins in the biological sample of formalin fixed tumor tissue;
wherein the one or more corresponding protein or proteins is selected from the group consisting of ABCG2, AIMP3, ALDH1B1, alpha-catenin, ARG2, ARID1A, ARID2, ATM, ATR, Bax, BCL2, Brachyury, BRCA1, BRE, CAD, Cav-1, CD8b, CD16, CD20, CD99, CD226, CDK4, CDK6, CDK10, CDX2, CHFR, CHK1, c-JUN, CLK4, COX41, COX5B, COX6C, DCTD, DDR1, DGKA, Diablo, DICER1, DUSP1, EIF3A, EMSY, EPCAM, ERCC2, FOXA1, FOXC1, FOXC2, FOXF2, gamma-catenin, GDA, GJA1, GLS, GLUT1, GSTM1, GSTM3, GSTP1, GSTT1, HAI2, hCNT3, HDAC6, HK2, HLA-A, HLA-B, HLA-DMA, HLA-DQA1, HLA-DRA, HPV16E6, HR23B, IDH1, IDH2, IGF2, IGF2BP3, IKZF1, IKZF3, IL13RA2, IL-15, IL-8, IRS2, LAG3, LGR5, LIG4, MCT1, MENA, MENAINV, MFF, MIG6, MLH1, MMP7, MMP9, MRE11, MSH2, MSH6, MUC4, ND1, NEDD8, Nibrin, NKp30, NKp44, NKp80, NNMT, NT5C, NT5C2, NT5C3A, P21, P27, PARP1, PARP16, PARP4, Paxillin, PDK1, PD-L2, PFKFB3, PI3KCA, PIM1, PIM2, PKM, PMS2, PSMA, PRKDC, QPRT, RAD50, SAMHD1, SIAH2, SIRT1, SLAMF6, SLC34A2, SLC46A1, SMARCA4, SMO, SOD1, SOD2, SOX9, SPRY2, TBX21, TJP1, TMSB15A, TPX2, TRIM24, TRKA, WT-1, XPO1, XRCC4, XRCC5, XRCC6, AMD1, FCER1G, RAD51C, PML, IL12RB2, SPAG5, NRAS, HRAS, MLH3, CEACAM6, LGALS9, RPA2, RPA1, FEN1, LIG3, BLM, B2M, RNPEP, IL12A, IL12B, YES1, NTRK1, NTRK2, NTRK3, and RB1.

2. The method of claim 1, further comprising the step of fractionating the protein digest prior to detecting and quantifying the amount of the one or more fragment peptides, wherein said fractionating step comprises liquid chromatography, nano-reverse phase liquid chromatography, high performance liquid chromatography or reverse phase high performance liquid chromatography.

3. The method of claim 1 or claim 2, wherein the mass spectrometry comprises tandem mass spectrometry, ion trap mass spectrometry, triple quadrupole mass spectrometry, hybrid ion trap/quadrupole mass spectrometry, MALDI-TOF mass spectrometry, MALDI mass spectrometry, and/or time of flight mass spectrometry.

4. The method of claim 3, wherein a mode of mass spectrometry used is Selected Reaction Monitoring (SRM), Multiple Reaction Monitoring (MRM), intelligent Selected Reaction Monitoring (iSRM), Parallel Reaction Monitoring (PRM), and/or multiple Selected Reaction Monitoring (mSRM).

5. The method of any one of the previous claims, wherein the one or more fragment peptides are selected from the group consisting of peptides corresponding to SEQ ID NO: 1-291.

6. The method of any one of the previous claims, wherein the formalin fixed tumor tissue is paraffin embedded tissue.

7. The method of any one of the previous claims, wherein quantifying the one or more fragment peptides comprises comparing an amount of the one or more fragment peptides in the biological sample to the amount of the same one or more fragment peptides in a different and separate biological sample.

8. The method of any one of the previous claims, wherein quantifying the one or more fragment peptides comprises determining an amount of the one or more fragment peptides in the biological sample by comparison to an added internal standard peptide of known amount having the same amino acid sequence of the one or more fragment peptides.

9. The method of claim 8, wherein the internal standard peptide is an isotopically labeled peptide, such as ¹⁸O, ¹⁷O, ³⁴S, ¹⁵N, ¹³C, ²H or a combination thereof.

10. The method of any one of the previous claims, wherein detecting and quantifying the amount of the one or more fragment peptides in the protein digest indicates the presence of the corresponding protein and an association with cancer in the subject.

11. The method of claim 10, further comprising correlating results of the detecting and quantifying the amount of the one or more fragment peptides, or the level of the corresponding protein to the activation status of the immune system of a cancer patient.

12. The method of claim 11, wherein the correlating results of the detecting and quantifying the amount of the one or more fragment peptides or the level of the corresponding protein to the activation status of the immune system of a cancer patient is combined with detecting and quantifying an amount of expression of other proteins or peptides from other proteins to provide additional information about a molecular status of tumor cells of the cancer patient.

13. The method of any one of the previous claims, further comprising selecting and determining a therapeutically effective amount of a cancer therapeutic agent which shall be administered to a patient or subject from which the biological sample was obtained,
wherein the cancer therapeutic agent and/or amount of the cancer therapeutic agent to be administered is based upon detection of and/or amount of the one or more fragment peptides selected from SEQ ID NO: 1-291, preferably multiplex detection of and/or amount of two or more fragment peptides selected from SEQ ID NO: 1-291, and
wherein the cancer therapeutic agent is a targeted agent that interacts with one or more proteins that correspond to the one or more fragment peptides selected from SEQ ID NO: 1-291.

14. The method of any one of the previous claims, further comprising selecting and determining a therapeutically effective amount of a cancer therapeutic agent which shall be administered to a patient or subject from which the biological sample was obtained,
wherein the cancer therapeutic agent and/or amount of the cancer therapeutic agent to be administered is based upon detection of and/or amount of the one or more fragment peptides selected from SEQ ID NO: 1-291, preferably multiplex detection of and/or amount of two or more fragment peptides selected from SEQ ID NO: 1-291, and
wherein the cancer therapeutic agent is an immunomodulatory cancer therapeutic agent whose function is to initiate, enhance, manipulate, and/or otherwise modulate the cancer patient immune response to attack and kill said patient tumor cells.

15. The method of any one of the previous claims, further comprising combining multiplex detecting and quantitating two or more fragment peptides corresponding to SEQ ID NO: 1-291 with analysis of other oncoproteins that drive growth of the patient tumor cells, wherein a targeted cancer therapeutic agent that inhibits or modulates the function of the oncoprotein to inhibit growth of the patient tumor cells is selected to be administered to the patient in combination with an immunomodulatory cancer therapeutic agent that interacts with one or more of the proteins to initiate, enhance, manipulate, and/or otherwise modulate the cancer patient immune response to attack and kill the patient tumor cells.


SEQUENCE LISTING

<110>  NantOmics, LLC

<120>  SRM/MRM Assays for Profiling Tumor Tissue

<130>  EP-21105056

<150>  US 62/640,632
<151>  2018-03-09

<160>  291

<170>  PatentIn version 3.5

<210>  1
<211>  10
<212>  PRT
<213>  Homo sapiens

<400>  1

Ser Ser Leu Leu Asp Val Leu Ala Ala Arg
1               5                   10


<210>  2
<211>  10
<212>  PRT
<213>  Homo sapiens

<400>  2

Thr Ile Ile Phe Ser Ile His Gln Pro Arg
1               5                   10


<210>  3
<211>  10
<212>  PRT
<213>  Homo sapiens

<400>  3

Ala Ile Val Gln Gln Trp Leu Glu Tyr Arg
1               5                   10


<210>  4
<211>  11
<212>  PRT
<213>  Homo sapiens

<400>  4

Glu Tyr Leu Leu Gly Ser Thr Ala Glu Glu Lys
1               5                   10


<210>  5
<211>  11
<212>  PRT
<213>  Homo sapiens

<400>  5

Leu Leu Asn Leu Leu Ala Asp Leu Val Glu Arg
1               5                   10


<210>  6
<211>  10
<212>  PRT
<213>  Homo sapiens

<400>  6

Tyr Gly Leu Ala Ala Ala Val Phe Thr Arg
1               5                   10


<210>  7
<211>  10
<212>  PRT
<213>  Homo sapiens

<400>  7

Ser Asp Ala Leu Asn Ser Ala Ile Asp Lys
1               5                   10


<210>  8
<211>  15
<212>  PRT
<213>  Homo sapiens

<400>  8

Ser Val Gly Leu Ala Asn Gln Glu Leu Ala Glu Val Val Ser Arg
1               5                   10                  15


<210>  9
<211>  12
<212>  PRT
<213>  Homo sapiens

<400>  9

Asp Asp Leu Tyr Asn Asn Leu Ile Val Asn Pro Arg
1               5                   10


<210>  10
<211>  7
<212>  PRT
<213>  Homo sapiens

<400>  10

Ala Leu Leu Ala Leu Ala Lys
1               5


<210>  11

<211>  9
<212>  PRT
<213>  Homo sapiens

<400>  11

Gly Leu Ala Phe Leu Asp Glu Leu Arg
1               5


<210>  12
<211>  10
<212>  PRT
<213>  Homo sapiens

<400>  12

Val Ala Ile Glu Ser Ala Val Gln Gln Lys
1               5                   10


<210>  13
<211>  10
<212>  PRT
<213>  Homo sapiens

<400>  13

Glu Leu Glu Leu Asp Glu Leu Ala Leu Arg
1               5                   10


<210>  14
<211>  10
<212>  PRT
<213>  Homo sapiens

<400>  14

Leu Val Val Asn Leu Leu Gln Leu Ser Lys
1               5                   10


<210>  15
<211>  13
<212>  PRT
<213>  Homo sapiens

<400>  15

Gly Glu Thr Phe Glu Val Pro Glu Ile Val Pro Phe Arg
1               5                   10


<210>  16
<211>  12
<212>  PRT
<213>  Homo sapiens

<400>  16

Thr Leu Gln Val Leu Leu Pro Asp Leu Ala Ala Lys
1               5                   10

<210>  17
<211>  5
<212>  PRT
<213>  Homo sapiens

<400>  17

Glu Val Phe Phe Arg
1               5


<210>  18
<211>  13
<212>  PRT
<213>  Homo sapiens

<400>  18

Thr Gly Ala Leu Leu Leu Gln Gly Phe Ile Gln Asp Arg
1               5                   10


<210>  19
<211>  10
<212>  PRT
<213>  Homo sapiens

<400>  19

Phe Ala Thr Val Val Glu Glu Leu Phe Arg
1               5                   10


<210>  20
<211>  11
<212>  PRT
<213>  Homo sapiens

<400>  20

Val Gly Leu Glu Glu Ser Glu Leu Trp Leu Arg
1               5                   10


<210>  21
<211>  18
<212>  PRT
<213>  Homo sapiens

<400>  21

Val Asp His Leu Leu Ser Ala Val Glu Asn Glu Leu Gln Ala Gly Ser
1               5                   10                  15


Glu Lys


<210>  22
<211>  8

<212>  PRT
<213>  Homo sapiens

<400>  22

Ile Pro Pro Leu Phe Pro Ile Lys
1               5


<210>  23
<211>  12
<212>  PRT
<213>  Homo sapiens

<400>  23

Val Gly Asn Ile Pro Ser Ser Thr Ser Ala Leu Lys
1               5                   10


<210>  24
<211>  14
<212>  PRT
<213>  Homo sapiens

<400>  24

Leu Pro Val Asp Phe Ser Asn Ile Pro Thr Tyr Leu Leu Lys
1               5                   10


<210>  25
<211>  6
<212>  PRT
<213>  Homo sapiens

<400>  25

Leu Tyr Leu Ser Pro Arg
1               5


<210>  26
<211>  14
<212>  PRT
<213>  Homo sapiens

<400>  26

Asn Thr Gly Val Ile Ser Val Val Thr Thr Gly Leu Asp Arg
1               5                   10


<210>  27
<211>  14
<212>  PRT
<213>  Homo sapiens

<400>  27

Tyr Val Asp Ser Glu Gly His Leu Tyr Thr Val Pro Ile Arg
1               5                   10

<210>  28
<211>  7
<212>  PRT
<213>  Homo sapiens

<400>  28

Ile Ser Leu Ser Asn Met Arg
1               5


<210>  29
<211>  11
<212>  PRT
<213>  Homo sapiens

<400>  29

Ala Val Val Phe Leu Glu Pro Gln Trp Tyr Arg
1               5                   10


<210>  30
<211>  12
<212>  PRT
<213>  Homo sapiens

<400>  30

Tyr Phe His His Asn Ser Asp Phe Tyr Ile Pro Lys
1               5                   10


<210>  31
<211>  11
<212>  PRT
<213>  Homo sapiens

<400>  31

Ser Asn Ile Val Leu Leu Ser Ala Glu Glu Lys
1               5                   10


<210>  32
<211>  14
<212>  PRT
<213>  Homo sapiens

<400>  32

Glu Glu Val Val Gly Leu Thr Glu Thr Ser Ser Gln Pro Lys
1               5                   10


<210>  33
<211>  10
<212>  PRT
<213>  Homo sapiens

<400>  33

Asn Ala Asn Ala Glu Pro Ala Val Gln Arg
1               5                   10


<210>  34
<211>  14
<212>  PRT
<213>  Homo sapiens

<400>  34

Glu Asn Ala Glu Gln Gly Glu Val Asp Met Glu Ser His Arg
1               5                   10


<210>  35
<211>  11
<212>  PRT
<213>  Homo sapiens

<400>  35

Asp Leu Phe Thr Glu Ser Trp Asp Thr Gln Lys
1               5                   10


<210>  36
<211>  12
<212>  PRT
<213>  Homo sapiens

<400>  36

Glu Asp Ile Tyr Val Asn Tyr Pro Thr Phe Ser Arg
1               5                   10


<210>  37
<211>  11
<212>  PRT
<213>  Homo sapiens

<400>  37

Asp Pro His Ser Gly His Phe Val Ala Leu Lys
1               5                   10


<210>  38
<211>  11
<212>  PRT
<213>  Homo sapiens

<400>  38

Leu Glu Ala Phe Glu His Pro Asn Val Val Arg
1               5                   10


<210>  39
<211>  15
<212>  PRT
<213>  Homo sapiens

<400>  39

Ile Leu Asp Val Ile Gly Leu Pro Gly Glu Glu Asp Trp Pro Arg
1               5                   10                  15


<210>  40
<211>  10
<212>  PRT
<213>  Homo sapiens

<400>  40

Phe Val Thr Asp Ile Asp Glu Leu Gly Lys
1               5                   10


<210>  41
<211>  8
<212>  PRT
<213>  Homo sapiens

<400>  41

Thr Ala Asp Phe Gly Leu Ala Arg
1               5


<210>  42
<211>  10
<212>  PRT
<213>  Homo sapiens

<400>  42

Phe Pro Trp Leu Ser Glu Ala Gly Leu Arg
1               5                   10


<210>  43
<211>  12
<212>  PRT
<213>  Homo sapiens

<400>  43

Ala Glu Leu Ala Ala Thr Leu Gly Leu Ser Glu Arg
1               5                   10


<210>  44
<211>  10
<212>  PRT
<213>  Homo sapiens

<400>  44

Asp Val Ser Met Tyr Pro Ser Ser Val Arg
1               5                   10


<210>  45

<211>  6
<212>  PRT
<213>  Homo sapiens

<400>  45

Glu Trp Thr Ile Gly Arg
1               5


<210>  46
<211>  9
<212>  PRT
<213>  Homo sapiens

<400>  46

Gly Val Phe Leu Leu Ser Asp Tyr Arg
1               5


<210>  47
<211>  11
<212>  PRT
<213>  Homo sapiens

<400>  47

Ile Ser Asp Phe Gly Leu Ala Thr Val Phe Arg
1               5                   10


<210>  48
<211>  9
<212>  PRT
<213>  Homo sapiens

<400>  48

Ile Leu Val Glu Asn Pro Ser Ala Arg
1               5


<210>  49
<211>  14
<212>  PRT
<213>  Homo sapiens

<400>  49

Asn Ser Asp Leu Leu Thr Ser Pro Asp Val Gly Leu Leu Lys
1               5                   10


<210>  50
<211>  15
<212>  PRT
<213>  Homo sapiens

<400>  50

Asn Val Thr Asp Glu Gln Glu Gly Phe Ala Glu Gly Phe Val Arg
1               5                   10                  15

<210>  51
<211>  6
<212>  PRT
<213>  Homo sapiens

<400>  51

Tyr Val Asp Glu Tyr Arg
1               5


<210>  52
<211>  14
<212>  PRT
<213>  Homo sapiens

<400>  52

Tyr Glu Ile Val Asp Thr Leu Gly Glu Gly Ala Phe Gly Lys
1               5                   10


<210>  53
<211>  10
<212>  PRT
<213>  Homo sapiens

<400>  53

Val Asn Pro Ile Gln Gly Leu Ala Ser Lys
1               5                   10


<210>  54
<211>  13
<212>  PRT
<213>  Homo sapiens

<400>  54

His Tyr Val Tyr Gly Pro Leu Pro Gln Ser Phe Asp Lys
1               5                   10


<210>  55
<211>  11
<212>  PRT
<213>  Homo sapiens

<400>  55

Gly Leu Asp Pro Tyr Asn Val Leu Ala Pro Lys
1               5                   10


<210>  56
<211>  8
<212>  PRT
<213>  Homo sapiens

<400>  56

Leu Val Pro Gln Gln Leu Ala His
1               5

<210>  57
<211>  7
<212>  PRT
<213>  Homo sapiens

<400>  57

Ala Tyr Ala Asp Phe Tyr Arg
1               5

<210>  58
<211>  8
<212>  PRT
<213>  Homo sapiens

<400>  58

Ala Gly Ile Phe Gln Ser Val Lys
1               5

<210>  59
<211>  8
<212>  PRT
<213>  Homo sapiens

<400>  59

Leu Ile Ile Gln Ala Gly Ile Lys
1               5

<210>  60
<211>  9
<212>  PRT
<213>  Homo sapiens

<400>  60

Ala Val Ser Val Pro Leu Gly Gly Arg
1               5

<210>  61
<211>  8
<212>  PRT
<213>  Homo sapiens

<400>  61

Asp Gly Pro Glu Ile Gly Leu Arg
1               5

<210>  62
<211>  8
<212>  PRT

<213>  Homo sapiens

<400>  62

Phe Gln Tyr Ile Leu Asn Pro Arg
1               5


<210>  63
<211>  7
<212>  PRT
<213>  Homo sapiens

<400>  63

Asn His Ile Gln Leu Val Lys
1               5


<210>  64
<211>  10
<212>  PRT
<213>  Homo sapiens

<400>  64

Leu Ala Glu Ala Gln Ile Glu Glu Leu Arg
1               5                   10


<210>  65
<211>  12
<212>  PRT
<213>  Homo sapiens

<400>  65

Leu Glu Asp Tyr Gln Asp Ala Val Ile Ile Pro Arg
1               5                   10


<210>  66
<211>  8
<212>  PRT
<213>  Homo sapiens

<400>  66

Phe Asp Leu Pro Thr Glu Tyr Arg
1               5


<210>  67
<211>  9
<212>  PRT
<213>  Homo sapiens

<400>  67

Leu Asp Glu Ala Phe Glu Phe Val Lys
1               5

<210>  68
<211>  8
<212>  PRT
<213>  Homo sapiens

<400>  68

Ala Ala Gln Val Phe Phe Leu Lys
1               5


<210>  69
<211>  13
<212>  PRT
<213>  Homo sapiens

<400>  69

Leu Ala Thr Leu Leu Gly Leu Gln Ala Pro Pro Thr Arg
1               5                   10


<210>  70
<211>  15
<212>  PRT
<213>  Homo sapiens

<400>  70

Leu Thr Ser Leu Val Pro Phe Val Asp Ala Phe Gln Leu Glu Arg
1               5                   10                  15


<210>  71
<211>  9
<212>  PRT
<213>  Homo sapiens

<400>  71

Leu Gln Gln Ala Pro Asn Gln Pro Lys
1               5


<210>  72
<211>  8
<212>  PRT
<213>  Homo sapiens

<400>  72

Gly Glu Ser Leu Phe His Ser Lys
1               5


<210>  73
<211>  6
<212>  PRT
<213>  Homo sapiens

<400>  73

Tyr Gln Leu Asp Pro Lys

1               5


<210>  74
<211>  10
<212>  PRT
<213>  Homo sapiens

<400>  74

Phe Tyr Glu Glu Phe Asp Ala His Gly Arg
1               5                   10


<210>  75
<211>  10
<212>  PRT
<213>  Homo sapiens

<400>  75

Thr Ala Glu His Phe Leu Gly Phe Leu Arg
1               5                   10


<210>  76
<211>  16
<212>  PRT
<213>  Homo sapiens

<400>  76

Asp Pro Ser Gly Ala Ser Asn Pro Ser Ala Asp Ser Pro Leu His Arg
1               5                   10                  15


<210>  77
<211>  8
<212>  PRT
<213>  Homo sapiens

<400>  77

Ala Gly Gly Gly Gly Asp Ala Lys
1               5


<210>  78
<211>  12
<212>  PRT
<213>  Homo sapiens

<400>  78

Ala Tyr Gly Pro Tyr Thr Pro Gln Pro Gln Pro Lys
1               5                   10


<210>  79
<211>  23
<212>  PRT
<213>  Homo sapiens

<400>  79

Gly Ser Pro Gln Ser Ala Ala Ala Glu Leu Ser Ser Gly Leu Leu Ala
1               5                   10                  15

Ser Ala Ala Ala Ser Ser Arg
            20

<210>  80
<211>  15
<212>  PRT
<213>  Homo sapiens

<400>  80

Val Glu Thr Leu Ser Pro Glu Ser Ala Leu Gln Gly Ser Pro Arg
1               5                   10                  15

<210>  81
<211>  8
<212>  PRT
<213>  Homo sapiens

<400>  81

Glu Pro Pro Pro Ala Ala Ser Lys
1               5

<210>  82
<211>  9
<212>  PRT
<213>  Homo sapiens

<400>  82

Glu Asp Leu Ser Val Gly Leu Pro Arg
1               5

<210>  83
<211>  12
<212>  PRT
<213>  Homo sapiens

<400>  83

Leu Leu Asn Asp Glu Asp Pro Val Val Val Thr Lys
1               5                   10

<210>  84
<211>  8
<212>  PRT
<213>  Homo sapiens

<400>  84

Phe Leu Tyr Leu Gly Asp Asp Arg
1               5

<210>  85
<211>  11
<212>  PRT
<213>  Homo sapiens

<400>  85

Asp His Leu Leu Gly Val Ser Asp Ser Gly Lys
1               5                   10


<210>  86
<211>  8
<212>  PRT
<213>  Homo sapiens

<400>  86

Ser Phe Pro Ile Ser His Val Arg
1               5


<210>  87
<211>  16
<212>  PRT
<213>  Homo sapiens

<400>  87

Gln Ala Ser Glu Gln Asn Trp Ala Asn Tyr Ser Ala Glu Gln Asn Arg
1               5                   10                  15


<210>  88
<211>  16
<212>  PRT
<213>  Homo sapiens

<400>  88

Val Ser Pro Glu Ser Asn Glu Asp Ile Ser Thr Thr Val Val Tyr Arg
1               5                   10                  15


<210>  89
<211>  18
<212>  PRT
<213>  Homo sapiens

<400>  89

His Ser Phe Gly Pro Leu Asp Tyr Glu Ser Leu Gln Gln Glu Leu Ala
1               5                   10                  15


Leu Lys


<210>  90
<211>  8

<212>  PRT
<213>  Homo sapiens

<400>  90

Val Thr Ile Leu Glu Leu Phe Arg
1               5


<210>  91
<211>  10
<212>  PRT
<213>  Homo sapiens

<400>  91

Thr Phe Asp Glu Ile Ala Ser Gly Phe Arg
1               5                   10


<210>  92
<211>  9
<212>  PRT
<213>  Homo sapiens

<400>  92

Phe Leu Pro Arg Pro Val Phe Ser Lys
1               5


<210>  93
<211>  8
<212>  PRT
<213>  Homo sapiens

<400>  93

Phe Ser Trp Phe Ala Gly Glu Lys
1               5


<210>  94
<211>  7
<212>  PRT
<213>  Homo sapiens

<400>  94

Ser Gln Trp Leu Asp Val Lys
1               5


<210>  95
<211>  7
<212>  PRT
<213>  Homo sapiens

<400>  95

Thr Leu Gly Leu Tyr Gly Lys
1               5

<210>  96
<211>  16
<212>  PRT
<213>  Homo sapiens

<400>  96

Phe Gln Asp Gly Asp Leu Thr Leu Tyr Gln Ser Asn Thr Ile Leu Arg
1               5                   10                  15


<210>  97
<211>  6
<212>  PRT
<213>  Homo sapiens

<400>  97

Ile Val Asp Leu Ile Lys
1               5


<210>  98
<211>  8
<212>  PRT
<213>  Homo sapiens

<400>  98

Asn Asp Ile Pro Phe Glu Leu Arg
1               5


<210>  99
<211>  7
<212>  PRT
<213>  Homo sapiens

<400>  99

Trp Tyr Phe Asp Val Glu Arg
1               5


<210>  100
<211>  12
<212>  PRT
<213>  Homo sapiens

<400>  100

Gln Gln Glu Asn Pro Pro Leu Pro Leu Gly Ser Lys
1               5                   10


<210>  101
<211>  8
<212>  PRT
<213>  Homo sapiens

<400>  101

Asp Ile Ala Ser Gly Ala Val Arg
1               5

<210>  102
<211>  13
<212>  PRT
<213>  Homo sapiens

<400>  102

Thr Asp Pro Gly Phe Ile Ala Phe Asp Trp Leu Gly Arg
1               5                   10

<210>  103
<211>  11
<212>  PRT
<213>  Homo sapiens

<400>  103

Glu Gln Leu Ile Gln Glu Gly Leu Leu Asp Arg
1               5                   10

<210>  104
<211>  9
<212>  PRT
<213>  Homo sapiens

<400>  104

Leu Glu Glu Leu Gly Leu Ala Gly Arg
1               5

<210>  105
<211>  11
<212>  PRT
<213>  Homo sapiens

<400>  105

Asn Val Glu Leu Val Glu Gly Glu Glu Gly Arg
1               5                   10

<210>  106
<211>  10
<212>  PRT
<213>  Homo sapiens

<400>  106

Leu Ser Pro Glu Leu Leu Asn Thr Gly Arg
1               5                   10

<210>  107
<211>  7
<212>  PRT
<213>  Homo sapiens

<400>  107

Val Asp Leu Gly Thr Leu Arg
1               5


<210>  108
<211>  6
<212>  PRT
<213>  Homo sapiens

<400>  108

Asp Tyr Ile Ala Leu Lys
1               5


<210>  109
<211>  14
<212>  PRT
<213>  Homo sapiens

<400>  109

Phe Ile Thr Val Gly Tyr Val Asp Asp Thr Leu Phe Val Arg
1               5                   10


<210>  110
<211>  10
<212>  PRT
<213>  Homo sapiens

<400>  110

Tyr Thr Ala Ile Ala Tyr Trp Val Pro Arg
1               5                   10


<210>  111
<211>  12
<212>  PRT
<213>  Homo sapiens

<400>  111

Val Glu His Trp Gly Leu Asp Lys Pro Leu Leu Lys
1               5                   10


<210>  112
<211>  6
<212>  PRT
<213>  Homo sapiens

<400>  112

Glu Asp His Leu Phe Arg
1               5


<210>  113

<211>  8
<212>  PRT
<213>  Homo sapiens

<400>  113

Glu Val Tyr Asp Phe Ala Phe Arg
1               5


<210>  114
<211>  12
<212>  PRT
<213>  Homo sapiens

<400>  114

Thr Ala Met Phe Gln Asp Pro Gln Glu Arg Pro Arg
1               5                   10


<210>  115
<211>  10
<212>  PRT
<213>  Homo sapiens

<400>  115

Ile Asp Ile Asp Pro Glu Glu Thr Val Lys
1               5                   10


<210>  116
<211>  9
<212>  PRT
<213>  Homo sapiens

<400>  116

Ile Leu Asn Asp Asp Thr Ala Leu Lys
1               5


<210>  117
<211>  7
<212>  PRT
<213>  Homo sapiens

<400>  117

Ile Ile Trp Glu Leu Ile Lys
1               5


<210>  118
<211>  13
<212>  PRT
<213>  Homo sapiens

<400>  118

Leu Val Ser Gly Trp Val Lys Pro Ile Ile Ile Gly Arg
1               5                   10

<210>  119
<211>  9
<212>  PRT
<213>  Homo sapiens

<400>  119

Tyr Phe Asp Leu Gly Leu Pro Asn Arg
1               5

<210>  120
<211>  13
<212>  PRT
<213>  Homo sapiens

<400>  120

Leu Val Pro Gly Trp Thr Lys Pro Ile Thr Ile Gly Arg
1               5                   10

<210>  121
<211>  8
<212>  PRT
<213>  Homo sapiens

<400>  121

Phe Phe Gln Tyr Asp Thr Trp Lys
1               5

<210>  122
<211>  7
<212>  PRT
<213>  Homo sapiens

<400>  122

Gly Leu Pro Ala Leu Leu Arg
1               5

<210>  123
<211>  10
<212>  PRT
<213>  Homo sapiens

<400>  123

Ile Ala Pro Ala Glu Ala Pro Asp Ala Lys
1               5                   10

<210>  124
<211>  8
<212>  PRT
<213>  Homo sapiens

<400>  124

Val Pro Ser Phe Ala Ala Gly Arg
1               5

<210>  125
<211>  14
<212>  PRT
<213>  Homo sapiens

<400>  125

Asp Gln Gly Ser Ser Ala Leu Ser Gly Val Gly Gly Ile Arg
1               5                   10

<210>  126
<211>  6
<212>  PRT
<213>  Homo sapiens

<400>  126

Ala Tyr Asp Leu Leu Arg
1               5

<210>  127
<211>  8
<212>  PRT
<213>  Homo sapiens

<400>  127

Glu Ser Glu Leu Ile Gln Thr Arg
1               5

<210>  128
<211>  7
<212>  PRT
<213>  Homo sapiens

<400>  128

Ser Ile His Leu Pro Glu Lys
1               5

<210>  129
<211>  11
<212>  PRT
<213>  Homo sapiens

<400>  129

Trp Ser Ile Pro Leu Gly Pro Ile Pro Ala Arg
1               5                   10

<210>  130
<211>  10
<212>  PRT

<213>  Homo sapiens

<400>  130

Phe Pro Tyr Leu Glu Ala Ser Asp Tyr Lys
1               5                   10


<210>  131
<211>  13
<212>  PRT
<213>  Homo sapiens

<400>  131

Thr Glu Ala Asn Trp Val Asn Val Ile Ser Asp Leu Lys
1               5                   10


<210>  132
<211>  6
<212>  PRT
<213>  Homo sapiens

<400>  132

Glu Asn Trp Val Gln Arg
1               5


<210>  133
<211>  10
<212>  PRT
<213>  Homo sapiens

<400>  133

Ala Leu Thr Asp Leu Val Ser Glu Gly Arg
1               5                   10


<210>  134
<211>  12
<212>  PRT
<213>  Homo sapiens

<400>  134

Thr Asp Ser Leu Ala Ala Thr Pro Pro Ala Ala Lys
1               5                   10


<210>  135
<211>  8
<212>  PRT
<213>  Homo sapiens

<400>  135

Leu Pro Ala Gly Val Gly Thr Arg
1               5

<210>  136
<211>  11
<212>  PRT
<213>  Homo sapiens

<400>  136

His Val Pro Thr Glu Ala Leu Gln Asn Leu Arg
1               5                   10


<210>  137
<211>  17
<212>  PRT
<213>  Homo sapiens

<400>  137

Ile Ala Ile Ile His Pro Asn Ala Phe Ser Thr Leu Pro Ser Leu Ile
1               5                   10                  15


Lys




<210>  138
<211>  8
<212>  PRT
<213>  Homo sapiens

<400>  138

Leu Ile Leu Pro Gln Leu Glu Arg
1               5


<210>  139
<211>  10
<212>  PRT
<213>  Homo sapiens

<400>  139

Leu Tyr Ile Glu Leu Leu Asn Leu Pro Arg
1               5                   10


<210>  140
<211>  12
<212>  PRT
<213>  Homo sapiens

<400>  140

Asp Leu His Asp Ala Asn Thr Asp Leu Ile Gly Arg
1               5                   10


<210>  141
<211>  7
<212>  PRT
<213>  Homo sapiens

<400>  141

Ser Ile Thr Val Phe Phe Lys
1               5


<210>  142
<211>  8
<212>  PRT
<213>  Homo sapiens

<400>  142

Leu Glu Gln Glu Gln Leu Glu Arg
1               5


<210>  143
<211>  7
<212>  PRT
<213>  Homo sapiens

<400>  143

Glu Gln Leu Glu Trp Glu Arg
1               5


<210>  144
<211>  8
<212>  PRT
<213>  Homo sapiens

<400>  144

Asn Gln Ile Val Phe Asp Asn Arg
1               5


<210>  145
<211>  11
<212>  PRT
<213>  Homo sapiens

<400>  145

Gly Ile Leu Ser Leu Ile Gln Ser Ser Thr Arg
1               5                   10


<210>  146
<211>  9
<212>  PRT
<213>  Homo sapiens

<400>  146

Leu Gln Leu Leu Glu Glu Glu Asn Lys
1               5


<210>  147

<211>  9
<212>  PRT
<213>  Homo sapiens

<400>  147

Glu Pro Leu Ser Pro Ser Asn Ser Arg
1               5


<210>  148
<211>  18
<212>  PRT
<213>  Homo sapiens

<400>  148

Val Ser Ser Thr His Tyr Tyr Leu Leu Pro Glu Arg Pro Pro Tyr Leu
1               5                   10                  15


Asp Lys



<210>  149
<211>  12
<212>  PRT
<213>  Homo sapiens

<400>  149

Ala Ile Glu Thr Val Tyr Ala Ala Tyr Leu Pro Lys
1               5                   10


<210>  150
<211>  7
<212>  PRT
<213>  Homo sapiens

<400>  150

Ile Leu Glu Val Val Gly Arg
1               5


<210>  151
<211>  9
<212>  PRT
<213>  Homo sapiens

<400>  151

Asp Leu Pro His Ile Thr Val Asp Arg
1               5


<210>  152
<211>  11
<212>  PRT
<213>  Homo sapiens

<400>  152

Ser Leu Gly Pro Ala Leu Leu Leu Leu Gln Lys
1               5                   10


<210>  153
<211>  9
<212>  PRT
<213>  Homo sapiens

<400>  153

Ala Val Ile Asp Asp Ala Phe Ala Arg
1               5


<210>  154
<211>  10
<212>  PRT
<213>  Homo sapiens

<400>  154

Ile Asp Ile Ser Pro Val Leu Leu Gln Lys
1               5                   10


<210>  155
<211>  10
<212>  PRT
<213>  Homo sapiens

<400>  155

Asn Val Gln Leu Ser Leu Leu Thr Glu Arg
1               5                   10


<210>  156
<211>  6
<212>  PRT
<213>  Homo sapiens

<400>  156

Asp Leu Leu Leu Val Arg
1               5


<210>  157
<211>  8
<212>  PRT
<213>  Homo sapiens

<400>  157

Gly Gly Ile Leu Ile Thr Glu Arg
1               5


<210>  158
<211>  9

<212>  PRT
<213>  Homo sapiens

<400>  158

Thr Ile Val Tyr Trp Gly Ile Gly Arg
1               5


<210>  159
<211>  9
<212>  PRT
<213>  Homo sapiens

<400>  159

Ile Ser Glu Val Val Glu Leu Leu Lys
1               5


<210>  160
<211>  14
<212>  PRT
<213>  Homo sapiens

<400>  160

Asn Asp Val Val Phe Gln Pro Ile Ser Gly Glu Asp Val Arg
1               5                   10


<210>  161
<211>  10
<212>  PRT
<213>  Homo sapiens

<400>  161

Ile Gly Leu Ala Ser Ala Leu Gln Pro Arg
1               5                   10


<210>  162
<211>  12
<212>  PRT
<213>  Homo sapiens

<400>  162

Thr Leu Leu Leu Thr Ser Leu Phe Leu Trp Ile Arg
1               5                   10


<210>  163
<211>  11
<212>  PRT
<213>  Homo sapiens

<400>  163

Glu Ile Glu Ile Asp Ile Glu Pro Thr Asp Lys
1               5                   10

<210>  164
<211>  10
<212>  PRT
<213>  Homo sapiens

<400>  164

Glu Glu Ser Leu Ala Asp Asp Leu Phe Arg
1               5                   10


<210>  165
<211>  9
<212>  PRT
<213>  Homo sapiens

<400>  165

Tyr Gly Thr Phe Val Asn Glu Glu Lys
1               5


<210>  166
<211>  8
<212>  PRT
<213>  Homo sapiens

<400>  166

Leu Ala Pro Leu Ala Ser Ser Arg
1               5


<210>  167
<211>  10
<212>  PRT
<213>  Homo sapiens

<400>  167

Leu Ala Ile Gly Ser Val Thr Trp Phe Arg
1               5                   10


<210>  168
<211>  9
<212>  PRT
<213>  Homo sapiens

<400>  168

Glu Ile Leu Tyr His Thr Val Ala Arg
1               5


<210>  169
<211>  15
<212>  PRT
<213>  Homo sapiens

<400>  169

Ala Gln Val Leu Gln Ser Val Ala Gly Gln Thr Leu Thr Val Arg
1               5                   10                  15

<210>  170
<211>  11
<212>  PRT
<213>  Homo sapiens

<400>  170

Ser Ser Ala Gln Thr Ser Gln Leu Thr Phe Lys
1               5                   10

<210>  171
<211>  10
<212>  PRT
<213>  Homo sapiens

<400>  171

Asp Tyr Ser Val Thr Leu His Trp Tyr Lys
1               5                   10

<210>  172
<211>  8
<212>  PRT
<213>  Homo sapiens

<400>  172

Phe Ser Ser Leu Pro Leu Gly Arg
1               5

<210>  173
<211>  8
<212>  PRT
<213>  Homo sapiens

<400>  173

Glu Ala Val Glu Ala Ala Val Lys
1               5

<210>  174
<211>  9
<212>  PRT
<213>  Homo sapiens

<400>  174

Leu Leu Ser Trp Ser Asp Asn Trp Arg
1               5

<210>  175
<211>  9
<212>  PRT
<213>  Homo sapiens

<400>  175

Asp Ala Val Asp Trp Val His Tyr Lys
1               5


<210>  176
<211>  10
<212>  PRT
<213>  Homo sapiens

<400>  176

Val Phe Leu Ala Thr Asn Ser Asp Tyr Lys
1               5                   10


<210>  177
<211>  9
<212>  PRT
<213>  Homo sapiens

<400>  177

Glu Gly Tyr Glu Asn Phe Phe Asp Lys
1               5


<210>  178
<211>  9
<212>  PRT
<213>  Homo sapiens

<400>  178

Gly Glu Leu Ile His Val Phe Asn Lys
1               5


<210>  179
<211>  18
<212>  PRT
<213>  Homo sapiens

<400>  179

Ser Gly Glu Gln Ala Glu Gly Ser Pro Gly Gly Pro Gly Asp Ser Gln
1               5                   10                  15


Gly Arg



<210>  180
<211>  8
<212>  PRT
<213>  Homo sapiens

<400>  180

Leu Tyr Leu Pro Thr Gly Pro Arg

1                   5


<210>  181
<211>  8
<212>  PRT
<213>  Homo sapiens

<400>  181

Tyr Glu Trp Gln Glu Val Glu Lys
1               5


<210>  182
<211>  14
<212>  PRT
<213>  Homo sapiens

<400>  182

Trp Asn Phe Asp Phe Gln Asn His Lys Pro Leu Glu Gly Lys
1               5                   10


<210>  183
<211>  13
<212>  PRT
<213>  Homo sapiens

<400>  183

Thr Thr Asn Phe Ala Gly Ile Leu Ser Gln Gly Leu Arg
1               5                   10


<210>  184
<211>  11
<212>  PRT
<213>  Homo sapiens

<400>  184

Thr Leu Gly Asp Phe Ala Ala Glu Tyr Ala Lys
1               5                   10


<210>  185
<211>  11
<212>  PRT
<213>  Homo sapiens

<400>  185

Asp Phe Glu Ala Leu Leu Ala Asp Ala Ser Lys
1               5                   10


<210>  186
<211>  10
<212>  PRT
<213>  Homo sapiens

<400>  186

Asp Leu Ile Tyr Ala Phe His Gly Ser Arg
1               5                   10


<210>  187
<211>  10
<212>  PRT
<213>  Homo sapiens

<400>  187

Ser Pro Val Asp Val Leu Gln Ile Phe Arg
1               5                   10


<210>  188
<211>  12
<212>  PRT
<213>  Homo sapiens

<400>  188

Ala Leu Asn Glu Asn Leu Gln Asp Thr Val Glu Lys
1               5                   10


<210>  189
<211>  8
<212>  PRT
<213>  Homo sapiens

<400>  189

Leu Gly Val Ala Thr Val Ala Lys
1               5


<210>  190
<211>  11
<212>  PRT
<213>  Homo sapiens

<400>  190

Glu Ile Ser Leu Leu Pro Asp Asn Leu Leu Arg
1               5                   10


<210>  191
<211>  16
<212>  PRT
<213>  Homo sapiens

<400>  191

Leu Tyr Ser Leu Glu Gly Tyr Gly Thr Asp Ala Val Ile Tyr Ile Lys
1               5                   10                  15


<210>  192
<211>  13

<212>  PRT
<213>  Homo sapiens

<400>  192

Thr Pro Glu Gly Leu Tyr Gln Val Thr Ser Val Leu Arg
1               5                   10


<210>  193
<211>  10
<212>  PRT
<213>  Homo sapiens

<400>  193

Ser Thr Ile Gln Thr Ala Glu Ala Leu Arg
1               5                   10


<210>  194
<211>  8
<212>  PRT
<213>  Homo sapiens

<400>  194

Val Phe Asn Val Gly Glu Tyr Arg
1               5


<210>  195
<211>  8
<212>  PRT
<213>  Homo sapiens

<400>  195

Val Ile Glu Pro Val Gly Asn Arg
1               5


<210>  196
<211>  6
<212>  PRT
<213>  Homo sapiens

<400>  196

Glu Ala Val Asp Leu Arg
1               5


<210>  197
<211>  10
<212>  PRT
<213>  Homo sapiens

<400>  197

Val Ser Asp Asn Leu Pro Val Ala Ile Lys
1               5                   10

<210>  198
<211>  10
<212>  PRT
<213>  Homo sapiens

<400>  198

Asp Glu Asn Ile Leu Ile Asp Leu Asn Arg
1               5                   10


<210>  199
<211>  7
<212>  PRT
<213>  Homo sapiens

<400>  199

Leu Gly Pro Leu Leu Gly Lys
1               5


<210>  200
<211>  9
<212>  PRT
<213>  Homo sapiens

<400>  200

Asp Glu Asn Ile Leu Ile Asp Leu Arg
1               5


<210>  201
<211>  14
<212>  PRT
<213>  Homo sapiens

<400>  201

Asp Pro Val Gln Glu Ala Trp Ala Glu Asp Val Asp Leu Arg
1               5                   10


<210>  202
<211>  9
<212>  PRT
<213>  Homo sapiens

<400>  202

Gly Asp Tyr Pro Leu Glu Ala Val Arg
1               5


<210>  203
<211>  7
<212>  PRT
<213>  Homo sapiens

<400>  203

Leu Leu Leu Ala Val Leu Lys
1               5

<210>  204
<211>  16
<212>  PRT
<213>  Homo sapiens

<400>  204

Gly Thr Thr Val Ser Val Gln Gln Leu Phe Ser Thr Leu Pro Val Arg
1               5                   10                  15

<210>  205
<211>  7
<212>  PRT
<213>  Homo sapiens

<400>  205

Ala Phe Leu Asp Glu Leu Lys
1               5

<210>  206
<211>  15
<212>  PRT
<213>  Homo sapiens

<400>  206

Ser Tyr Pro Asp Gly Trp Asn Leu Pro Gly Gly Gly Val Gln Arg
1               5                   10                  15

<210>  207
<211>  8
<212>  PRT
<213>  Homo sapiens

<400>  207

Leu Leu Glu Glu Ala Leu Leu Arg
1               5

<210>  208
<211>  11
<212>  PRT
<213>  Homo sapiens

<400>  208

Asp Gln Asn Ile Leu Leu Gly Thr Thr Tyr Arg
1               5                   10

<210>  209
<211>  8
<212>  PRT
<213>  Homo sapiens

<400>  209

Val Ala Leu Asn Thr Leu Ala Arg
1               5


<210>  210
<211>  12
<212>  PRT
<213>  Homo sapiens

<400>  210

Tyr Gly Leu Leu Val Gly Gly Ala Ala Ser His Arg
1               5                   10


<210>  211
<211>  9
<212>  PRT
<213>  Homo sapiens

<400>  211

Ser Glu Leu Leu Val Glu Gln Gly Arg
1               5


<210>  212
<211>  9
<212>  PRT
<213>  Homo sapiens

<400>  212

Phe Asp Glu Ile Phe Ser Ala Thr Arg
1               5


<210>  213
<211>  12
<212>  PRT
<213>  Homo sapiens

<400>  213

Phe Glu Asn Leu Gly Val Ser Ser Leu Gly Glu Arg
1               5                   10


<210>  214
<211>  12
<212>  PRT
<213>  Homo sapiens

<400>  214

Tyr Val Gly Glu Thr Gln Pro Thr Gly Gln Ile Lys
1               5                   10


<210>  215

<211>  8
<212>  PRT
<213>  Homo sapiens

<400>  215

Gly Ala Leu Thr Pro Ser Ile Arg
1               5


<210>  216
<211>  7
<212>  PRT
<213>  Homo sapiens

<400>  216

Leu Glu Leu Asn Gly Asn Arg
1               5


<210>  217
<211>  10
<212>  PRT
<213>  Homo sapiens

<400>  217

Gly Asp Ile Phe Asn Gln Val Val Pro Arg
1               5                   10


<210>  218
<211>  17
<212>  PRT
<213>  Homo sapiens

<400>  218

Asn Tyr Thr Gln Asn Ile Asp Thr Leu Glu Gln Val Ala Gly Ile Gln
1               5                   10                  15


Arg



<210>  219
<211>  9
<212>  PRT
<213>  Homo sapiens

<400>  219

Asp Ser Leu Ser Leu Ser Thr Gln Arg
1               5


<210>  220
<211>  8
<212>  PRT
<213>  Homo sapiens

<400>  220

Glu Thr Glu Ile Trp Thr Pro Arg
1               5


<210>  221
<211>  12
<212>  PRT
<213>  Homo sapiens

<400>  221

Glu Ala Gln Gly Glu Val Pro Ala Ser Asp Ser Lys
1               5                   10


<210>  222
<211>  12
<212>  PRT
<213>  Homo sapiens

<400>  222

Ala Pro Glu Thr Phe Asp Asn Ile Thr Ile Ser Arg
1               5                   10


<210>  223
<211>  13
<212>  PRT
<213>  Homo sapiens

<400>  223

Ser Ile Val Gln Leu Tyr Val Ala Pro Ala Pro Glu Lys
1               5                   10


<210>  224
<211>  11
<212>  PRT
<213>  Homo sapiens

<400>  224

Asp Thr Ala Leu Glu Thr Ala Leu Asn Ala Lys
1               5                   10


<210>  225
<211>  10
<212>  PRT
<213>  Homo sapiens

<400>  225

Gly Val Leu Leu Thr Asp Gly Ser Glu Lys
1               5                   10


<210>  226
<211>  8

<212>  PRT
<213>  Homo sapiens

<400>  226

Leu Val Leu Trp Ser Gly Leu Arg
1               5


<210>  227
<211>  7
<212>  PRT
<213>  Homo sapiens

<400>  227

Gly Trp Pro Asp Phe Leu Arg
1               5


<210>  228
<211>  14
<212>  PRT
<213>  Homo sapiens

<400>  228

Gly Asp Gly Pro Val Gln Gly Ile Ile Asn Phe Glu Gln Lys
1               5                   10


<210>  229
<211>  12
<212>  PRT
<213>  Homo sapiens

<400>  229

His Val Gly Asp Leu Gly Asn Val Thr Ala Asp Lys
1               5                   10


<210>  230
<211>  14
<212>  PRT
<213>  Homo sapiens

<400>  230

Ala Ile Trp Asn Val Ile Asn Trp Glu Asn Val Thr Glu Arg
1               5                   10


<210>  231
<211>  7
<212>  PRT
<213>  Homo sapiens

<400>  231

Tyr Gln Glu Ala Leu Ala Lys
1               5

<210>  232
<211>  8
<212>  PRT
<213>  Homo sapiens

<400>  232

Glu Ala Val Ser Gln Val Leu Lys
1               5


<210>  233
<211>  17
<212>  PRT
<213>  Homo sapiens

<400>  233

Leu Leu Gly Ser Ser Phe Ser Ser Gly Pro Val Ala Asp Gly Ile Ile
1               5                   10                  15


Arg




<210>  234
<211>  8
<212>  PRT
<213>  Homo sapiens

<400>  234

Leu His Gly Leu Pro Glu His Arg
1               5


<210>  235
<211>  10
<212>  PRT
<213>  Homo sapiens

<400>  235

Phe Tyr Pro Asp Leu Pro Gly Gln Ala Lys
1               5                   10


<210>  236
<211>  14
<212>  PRT
<213>  Homo sapiens

<400>  236

Tyr Phe Tyr Pro Glu Pro Gly Ala Gln Asp Ala Asp Glu Arg
1               5                   10


<210>  237
<211>  8
<212>  PRT

<213>  Homo sapiens

<400>  237

Leu Gly Ser Trp Leu Ala Ile Arg
1               5


<210>  238
<211>  11
<212>  PRT
<213>  Homo sapiens

<400>  238

Glu Gly Leu Glu Glu Gly Asp Gln Ile Leu Arg
1               5                   10


<210>  239
<211>  7
<212>  PRT
<213>  Homo sapiens

<400>  239

Glu Thr Ile Gln Gln Glu Lys
1               5


<210>  240
<211>  9
<212>  PRT
<213>  Homo sapiens

<400>  240

Ser Thr Glu Glu Gln Glu Leu Glu Lys
1               5


<210>  241
<211>  11
<212>  PRT
<213>  Homo sapiens

<400>  241

Leu Ala Leu Ala Gly Ile Gly Gln Pro Val Lys
1               5                   10


<210>  242
<211>  9
<212>  PRT
<213>  Homo sapiens

<400>  242

Phe Thr Gly Asn Gln Ile Gln Asn Arg
1               5

<210> 243
<211> 12
<212> PRT
<213> Homo sapiens

<400> 243

Asp Thr Thr Glu Val Pro Ser Ser Thr Val Glu Lys
1               5                   10


<210> 244
<211> 10
<212> PRT
<213> Homo sapiens

<400> 244

Trp Glu Leu Gly Glu Gly Ala Phe Gly Lys
1               5                   10


<210> 245
<211> 10
<212> PRT
<213> Homo sapiens

<400> 245

Val Pro Gly Val Ala Pro Thr Leu Val Arg
1               5                   10


<210> 246
<211> 9
<212> PRT
<213> Homo sapiens

<400> 246

Asp Thr Asp Ser Ile Asn Leu Tyr Lys
1               5


<210> 247
<211> 12
<212> PRT
<213> Homo sapiens

<400> 247

Ala Val Gly His Pro Phe Val Ile Gln Leu Gly Arg
1               5                   10


<210> 248
<211> 9
<212> PRT
<213> Homo sapiens

<400> 248

Val Glu Asn Pro Ala Glu Val Ile Arg

1               5


<210>  249
<211>  9
<212>  PRT
<213>  Homo sapiens

<400>  249

Glu Ala Leu Glu Thr Asp Leu Tyr Lys
1               5


<210>  250
<211>  8
<212>  PRT
<213>  Homo sapiens

<400>  250

Glu Asp Ile Ile Gln Gly Phe Arg
1               5


<210>  251
<211>  7
<212>  PRT
<213>  Homo sapiens

<400>  251

Glu Gly Leu Glu Ile Val Lys
1               5


<210>  252
<211>  11
<212>  PRT
<213>  Homo sapiens

<400>  252

Asp Ser Leu Ile Phe Leu Val Asp Ala Ser Lys
1               5                   10


<210>  253
<211>  14
<212>  PRT
<213>  Homo sapiens

<400>  253

Asn Ile Tyr Val Leu Gln Glu Leu Asp Asn Pro Gly Ala Lys
1               5                   10


<210>  254
<211>  7
<212>  PRT
<213>  Homo sapiens

<400>  254

Ala Leu Val Pro Leu Leu Lys
1               5


<210>  255
<211>  8
<212>  PRT
<213>  Homo sapiens

<400>  255

Ser Glu Trp Asp Ile Leu Leu Lys
1               5


<210>  256
<211>  11
<212>  PRT
<213>  Homo sapiens

<400>  256

Ser Asp Gly Val Tyr Thr Gly Leu Ser Thr Arg
1               5                   10


<210>  257
<211>  8
<212>  PRT
<213>  Homo sapiens

<400>  257

Ala Ala Ile Thr Ser Tyr Glu Lys
1               5


<210>  258
<211>  11
<212>  PRT
<213>  Homo sapiens

<400>  258

Ala Glu Ala Leu Glu Thr Leu Gln Ile Ile Arg
1               5                   10


<210>  259
<211>  7
<212>  PRT
<213>  Homo sapiens

<400>  259

Leu Ile Phe His Trp Asp Arg
1               5


<210>  260
<211>  9

<212>  PRT
<213>  Homo sapiens

<400>  260

Glu Leu Leu Glu Ala Val Asp Ala Arg
1               5


<210>  261
<211>  7
<212>  PRT
<213>  Homo sapiens

<400>  261

Leu Asp Ala Val Leu Gln Arg
1               5


<210>  262
<211>  15
<212>  PRT
<213>  Homo sapiens

<400>  262

Ile Leu His Tyr Gln Val Thr Leu Gln Glu Leu Thr Gly Gly Lys
1               5                   10                  15


<210>  263
<211>  8
<212>  PRT
<213>  Homo sapiens

<400>  263

Ser Gly Glu Leu Ile Ser Leu Arg
1               5


<210>  264
<211>  10
<212>  PRT
<213>  Homo sapiens

<400>  264

Thr Leu Leu Ser Ile Pro Glu Val Val Arg
1               5                   10


<210>  265
<211>  9
<212>  PRT
<213>  Homo sapiens

<400>  265

Ser Phe Ala Asp Ile Asn Leu Tyr Arg
1               5

<210>  266
<211>  9
<212>  PRT
<213>  Homo sapiens

<400>  266

Ser Phe Glu Asp Ile His Gln Tyr Arg
1               5


<210>  267
<211>  12
<212>  PRT
<213>  Homo sapiens

<400>  267

Leu Ser Leu Ser Ser Gln Leu Gly Ser Leu Glu Lys
1               5                   10


<210>  268
<211>  13
<212>  PRT
<213>  Homo sapiens

<400>  268

Glu Val Leu Leu Leu Ala His Asn Leu Pro Gln Asn Arg
1               5                   10


<210>  269
<211>  7
<212>  PRT
<213>  Homo sapiens

<400>  269

Ile Gly Tyr Ser Trp Tyr Lys
1               5


<210>  270
<211>  14
<212>  PRT
<213>  Homo sapiens

<400>  270

Ser Ile Leu Leu Ser Gly Thr Val Leu Pro Ser Ala Gln Arg
1               5                   10


<210>  271
<211>  12
<212>  PRT
<213>  Homo sapiens

<400>  271

Ile Asp Asp Met Thr Ala Ala Pro Met Asp Val Arg
1               5                   10


<210>  272
<211>  10
<212>  PRT
<213>  Homo sapiens

<400>  272

Ala Thr Ala Phe Asn Glu Gln Val Asp Lys
1               5                   10


<210>  273
<211>  14
<212>  PRT
<213>  Homo sapiens

<400>  273

Leu Phe Ser Leu Glu Leu Val Asp Glu Ser Gly Glu Ile Arg
1               5                   10


<210>  274
<211>  11
<212>  PRT
<213>  Homo sapiens

<400>  274

Leu Ile Ala Asp Val Ala Pro Ser Ala Ile Arg
1               5                   10


<210>  275
<211>  11
<212>  PRT
<213>  Homo sapiens

<400>  275

Trp Ser Glu Pro Asn Glu Glu Glu Leu Ile Lys
1               5                   10


<210>  276
<211>  10
<212>  PRT
<213>  Homo sapiens

<400>  276

Val Leu Leu Asp Ile Phe Thr Gly Val Arg
1               5                   10


<210>  277
<211>  12
<212>  PRT
<213>  Homo sapiens

<400>  277

Asp Leu Glu Gln Gly Asp Val Ser Glu Thr Ile Arg
1               5                   10


<210>  278
<211>  9
<212>  PRT
<213>  Homo sapiens

<400>  278

Leu Leu Thr Glu Val Asp Phe Asn Lys
1               5


<210>  279
<211>  10
<212>  PRT
<213>  Homo sapiens

<400>  279

Val Asn His Val Thr Leu Ser Gln Pro Lys
1               5                   10


<210>  280
<211>  10
<212>  PRT
<213>  Homo sapiens

<400>  280

Val Glu His Ser Asp Leu Ser Phe Ser Lys
1               5                   10


<210>  281
<211>  13
<212>  PRT
<213>  Homo sapiens

<400>  281

Ala Glu Phe Gly Pro Pro Gly Pro Gly Ala Gly Ser Arg
1               5                   10


<210>  282
<211>  11
<212>  PRT
<213>  Homo sapiens

<400>  282

Val Asp Ile Ile Pro Gly Phe Glu Phe Asp Arg
1               5                   10


<210>  283

<211>  10
<212>  PRT
<213>  Homo sapiens

<400>  283

Ser Ser Leu Glu Glu Pro Asp Phe Tyr Lys
1               5                   10


<210>  284
<211>  7
<212>  PRT
<213>  Homo sapiens

<400>  284

Thr Leu Thr Ile Gln Val Lys
1               5


<210>  285
<211>  7
<212>  PRT
<213>  Homo sapiens

<400>  285

Asn Ala Ser Ile Ser Val Arg
1               5


<210>  286
<211>  8
<212>  PRT
<213>  Homo sapiens

<400>  286

Ala Gln Phe Asp Thr Leu Gln Lys
1               5


<210>  287
<211>  9
<212>  PRT
<213>  Homo sapiens

<400>  287

Leu Leu Leu Asn Pro Gly Asn Gln Arg
1               5


<210>  288
<211>  8
<212>  PRT
<213>  Homo sapiens

<400>  288

Ile Gly Asp Phe Gly Met Ser Arg
1               5

<210>  289
<211>  8
<212>  PRT
<213>  Homo sapiens

<400>  289

Ile Gly Asp Phe Gly Met Ser Arg
1               5


<210>  290
<211>  8
<212>  PRT
<213>  Homo sapiens

<400>  290

Ile Gly Asp Phe Gly Met Ser Arg
1               5


<210>  291
<211>  13
<212>  PRT
<213>  Homo sapiens

<400>  291

Phe Asp Ile Glu Gly Ser Asp Glu Ala Asp Gly Ser Lys
1               5                   10